(19) Europäisches Patentamt
European Patent Office
Office européen des brevets

(11) **EP 3 119 361 B1**

## (12) EUROPEAN PATENT SPECIFICATION

(45) Date of publication and mention of the grant of the patent:
**13.06.2018 Bulletin 2018/24**

(21) Application number: **15715906.2**

(22) Date of filing: **20.03.2015**

(51) Int Cl.:
*A61F 13/15* *(2006.01)*   *D04H 1/26* *(2012.01)*
*D04H 1/4266* *(2012.01)*

(86) International application number:
**PCT/US2015/021824**

(87) International publication number:
**WO 2015/143364 (24.09.2015 Gazette 2015/38)**

### (54) SPUNBOND WEB MATERIAL WITH IMPROVED TACTILE SOFTNESS ATTRIBUTES

SPINNBAHNMATERIAL MIT VERBESSERTEN TAKTILEN WEICHHEITSEIGENSCHAFTEN

MATÉRIAU DE TYPE VOILE NON-TISSÉ PRÉSENTANT DES CARACTÉRISTIQUES AMÉLIORÉES EN MATIÈRE DE DOUCEUR AU TOUCHER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.03.2014 US 201461968415 P**

(43) Date of publication of application:
**25.01.2017 Bulletin 2017/04**

(73) Proprietor: **The Procter & Gamble Company Cincinnati, OH 45202 (US)**

(72) Inventor: **KANYA, Kevin, Ronald Cincinnati, Ohio 45202 (US)**

(74) Representative: **Heide, Ute Procter & Gamble Service GmbH IP Department Sulzbacher Straße 40-50 65824 Schwalbach am Taunus (DE)**

(56) References cited:
**WO-A1-2012/024576    WO-A1-2012/130414 WO-A1-2014/044235    US-A1- 2006 057 921**

**Description**

BACKGROUND OF THE INVENTION

The business of manufacturing and marketing disposable absorbent articles for personal care or hygiene (such as disposable diapers, training pants, adult incontinence undergarments, feminine hygiene products, breast pads, care mats, bibs, wound dressing products, and the like) is relatively capital intensive and highly competitive. To maintain or grow their market share and thereby maintain a successful business, manufacturers of such articles must continually strive to enhance their products in ways that serve to differentiate them from those of their competitors, while at the same time controlling costs so as to enable competitive pricing and the offering to the market of an attractive value-to-price proposition.

One way in which some manufacturers may seek to enhance such products is through enhancements to softness. Parents and caregivers naturally seek to provide as much comfort as they can for their babies, and utilizing products such as disposable diapers that they perceive as relatively soft provides reassurance that they are doing what they can to provide comfort in that context. With respect to other types of disposable absorbent articles that are designed to be applied and/or worn close the skin, an appearance of softness can reassure the wearer or caregiver that the article will be comfortable.

Thus, manufacturers may devote efforts toward enhancing the softness of the various materials used to make such products, such as various web materials, including nonwoven web materials formed from polymer fibers, and laminates thereof, forming the products. Such laminates may include, for example, laminates of polymer films and nonwoven web materials forming the backsheet components of the products.

It is believed that humans' perceptions of softness of a nonwoven web material can be affected by tactile signals, auditory signals and visual signals.

Tactile softness signals may be affected by a variety of the material's features and properties that have effect on its tactile feel, including but not limited to loft, fiber thickness and density, basis weight, microscopic pliability and flexibility of individual fibers, macroscopic pliability and flexibility of the nonwoven web as formed by the fibers, surface friction characteristics, number of loose fibers or free fiber ends, and other features.

Perceptions of softness also may be affected by auditory signals, *e.g.*, whether and to what extent the material makes audible rustling, crinkling or other noises when touched or manipulated.

It is believed that perceptions of softness of a material also may be affected by visual signals, *i.e.*, its visual appearance. It is believed that, if a nonwoven material *looks* relatively soft to a person, it is much more likely that the person will perceive it as having relative tactile softness as well. Visual impressions of softness may be affected by a variety of features and properties, including but not limited to color, opacity, light reflectivity, refractivity or absorption, apparent thickness/caliper, fiber size and density, and macroscopic physical surface features.

As a result of the complexity of the mix of the above-described characteristics, to the extent softness is considered an attribute of a nonwoven web material, it may elude precise measurement or quantification. Although several methods for measuring and evaluating material features that are believed to affect softness signals have been developed, there are no standard, universally accepted units or methods of measurement for softness. It is a subjective, relative concept, difficult to characterize in an objective way. Because softness is difficult to characterize, it can also be difficult to affect in a predictable way, through changes or adjustments to specifications in materials or manufacturing processes.

Complicating efforts to define and enhance softness is the fact that differing individuals will have differing individual physiological and experiential frames of reference and perceptions concerning what material features and properties will cause them to perceive softness to a lesser or greater extent in a material, and relative other materials.

Various efforts have been made to provide or alter features of nonwoven web materials with the objective of enhancing consumer perceptions of softness. These efforts have included selection and/or manipulation of fiber chemistry, basis weight, loft, fiber density, configuration and size, tinting and/or opacifying, embossing or bonding in various patterns, etc.

These approaches have had varying degrees of success, but have left room for improvement in enhancing visual and/or tactile softness signals.

US 2006/057921 relates to a hydroengorged spunmelt nonwoven formed of thermoplastic continuous fibers and a pattern of fusion bonds. The nonwoven has either a percentage bond area of less than 10 percent, or a percentage bond area of at least 10% wherein the pattern of fusion bonds is anisotropic.

WO 2014/044235 discloses a nonwoven web comprising heat bondable fibres and comprising a plurality of calendering bonds having a bond shape. The heat bondable fibres comprise propylene copolymer, softness enhanced additive and polypropylene. The plurality of calendering bonds having a bond shape forms a regular pattern. The bond shapes have a greatest measurable length and a greatest measurable width, wherein an aspect ratio of the greatest measurable length to the greatest measurable width is at least 2.5.

WO 2012/130414 refers to a process of forming a soft bulky nonwoven web from a batt using thermobonding and to a soft bulky nonwoven web with a bond impression pattern and shape.

WO 2012/024576 relates to an absorbent article having improved softness signals. The web may be formed of spunlaid fibers including polyolefin and up to 5 percent by weight $TiO_2$, and may be impressed with a pattern of bond impressions to a bond area percentage of at least 10 percent forming a pattern of bonded regions and raised regions. The web may have opacity of 42 or greater; have an average height difference between bonded regions and raised regions of at least 280 $\mu$m; be hydroengorged; and/or have a cross-direction tensile strength of 350 gf/cm.

BRIEF DESCRIPTION OF THE DRAWINGS

Fig. 1A is a perspective view of a disposable diaper shown laid out horizontally in a relaxed state, wearer-facing surfaces up;
Fig. 1B is a plan view of a disposable diaper shown laid out horizontally in a stretched out, flattened state (stretched out against elastic contraction induced by the presence of elastic members), wearer-facing surfaces facing the viewer;
Fig. 2A is a cross section of the diaper depicted in Figs. 1A and 1B, taken through line 2-2 in those figures;
Fig. 2B is a schematic cross section of a portion of a laminate of a polymeric film and a nonwoven web, taken through a path of bond impressions;
Fig. 3A is a schematic depiction of a pattern(s) that may be machined, etched, engraved or otherwise formed on the working surface of a calender-bonding roller;
Fig. 3B is a schematic depiction of a pattern(s) of bond impressions that may be impressed on a nonwoven web;
Fig. 4A is an image of a nonwoven web sample taken using equipment described in the Average Measured Height Method set forth herein, illustrating an outline of an unbonded area; and
Fig. 4B is an image of a nonwoven web sample taken using equipment described in the Average Measured Height Method set forth herein, illustrating outlines of individual bond impressions.

DETAILED DESCRIPTION OF THE INVENTION

Definitions

"Absorbent article" refers to devices that absorb and contain body exudates, and, more specifically, refers to devices that are placed against or in proximity to the body of the wearer to absorb and contain the various exudates discharged from the body. Absorbent articles may include diapers, training pants, adult incontinence undergarments and pads, feminine hygiene products, breast pads, care mats, bibs, wound dressing products, and the like. As used herein, the term "exudates" includes, but is not limited to, urine, blood, vaginal discharges, breast milk, sweat and fecal matter.

"Absorbent core" means a structure typically disposed between a topsheet and backsheet of an absorbent article for absorbing and containing liquid received by the absorbent article. The absorbent core may also include a cover layer or envelope. The cover layer or envelope may comprise a nonwoven. In some examples, the absorbent core may include one or more substrates, an absorbent polymer material, and a thermoplastic adhesive material/composition adhering and immobilizing the absorbent polymer material to a substrate, and optionally a cover layer or envelope.

"Absorbent polymer material," "absorbent gelling material," "AGM," "superabsorbent," and "superabsorbent material" are used herein interchangeably and refer to cross linked polymeric materials that can absorb at least 5 times their weight of an aqueous 0.9% saline solution as measured using the Centrifuge Retention Capacity test (Edana 441.2-01).

"Absorbent particulate polymer material" is used herein to refer to an absorbent polymer material which is in particulate form so as to be flowable in the dry state.

"Absorbent particulate polymer material area" as used herein refers to the area of the core wherein the first substrate and second substrate are separated by a multiplicity of superabsorbent particles. There may be some extraneous superabsorbent particles outside of this area between the first substrate 64 and second substrate.

"Airfelt" is used herein to refer to comminuted wood pulp, which is a form of cellulosic fiber.

"Average Measured Height" is an average difference in z-direction height between raised, unbonded areas, and bond impressions, of a nonwoven web component of a laminate of a polymeric film and a nonwoven web, measured and calculated according to the Average Measured Height Method set forth herein.

A "batt" is used herein to refer to fiber materials prior to being consolidated in a final calendering process as described herein. A "batt" comprises individual fibers, which are usually unbonded to each other, although a certain amount of pre-bonding between fibers may be performed and is also included in the meaning, such as may occur during or shortly after the lay-down of fibers in a spunlaying process, or as may be achieved be a pre-calendering. This pre-bonding, however, still permits a substantial number of the fibers to be freely moveable such that they can be repositioned. A "batt" may comprise several strata, such as may result from depositing fibers from several beams in a spunlaying process.

"Bicomponent" refers to fiber having a cross-section comprising two discrete polymer components, two discrete blends of polymer components, or one discrete polymer component and one discrete blend of polymer components. "Bicomponent fiber" is encompassed within the term "Multicomponent fiber." A Bicomponent fiber may have an overall cross section divided into two or more subsections of the differing components of any shape or arrangement, including, for example, coaxial subsections, core-and-sheath subsections, side-by-side subsections, radial subsections, etc.

"Bond Area Percentage" on a nonwoven web is a ratio of area occupied by bond impressions, to the total surface area of the web, expressed as a percentage, and measured according to the Bond Area Percentage method set forth herein.

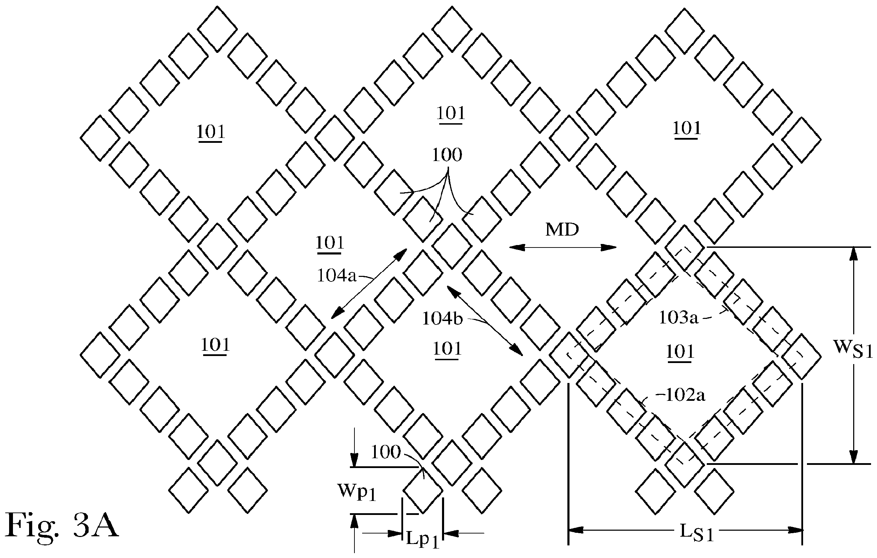

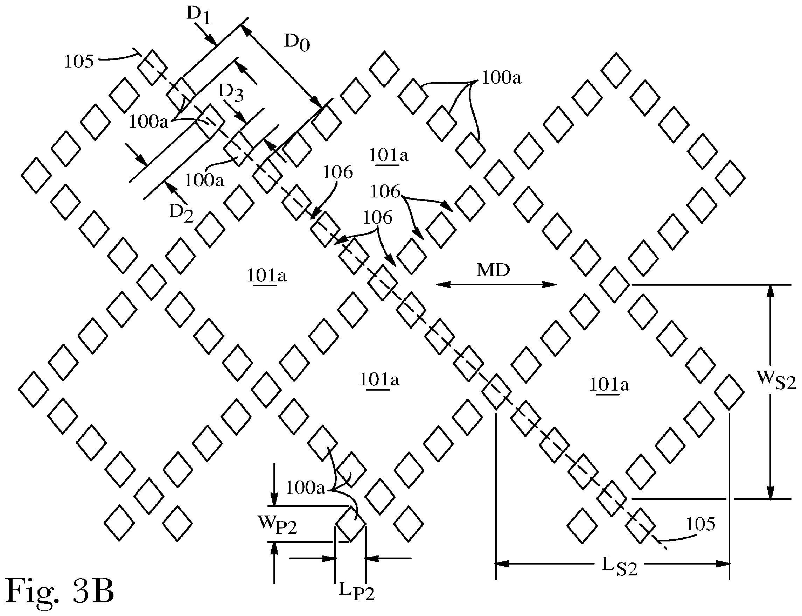

"Bond Length Ratio" is a value expressed as percentage, and is the ratio of the sum of lengths of a repeating series of bond impressions on a nonwoven web along a theoretical line segment through and connecting the bond impressions in the series, and extending from a leading edge of the bond impression beginning the series, to a leading edge of the bond impression beginning the next adjacent repeating series, to the total length of the line segment, and is determined according to the Bond Path / Bond Length Ratio measurement method set forth herein. By way of non-limiting illustration Fig. 3B in which length $D_0$ is the length of a line segment and lengths $D_1$, $D_2$ and $D_3$ are lengths along the line segment of three bond impressions in a hypothetical repeating series of substantially identical bond impressions 100a as shown in Fig. 3B, a Bond Length Ratio may be calculated as $[(D_1 + D_2 + D_3)/ D_0]$ x 100%. It will be noted that if all bond impressions 100a as exemplified in Fig. 3B are identical in area, shape and spacing, any group of them in any number along a line segment will constitute a repeating series. However, bond impressions forming a path also may have differing areas, shapes and/or spacing, and it may be necessary to identify a repeating series of bond impressions of any other particular number in order to determine Bond Length Ratio.

"Bonding roller," "calender roller" and "roller" are used interchangeably.

A "bond impression" in a nonwoven web is the surface structure created by the impression of a bonding protrusion on a calender roller into a nonwoven web. A bond impression is a location of deformed, intermeshed or entangled, and melted or thermally fused, materials from fibers superimposed and compressed in a z-direction beneath the bonding protrusion, which form a bond. The individual bonds may be connected in the nonwoven structure by loose fibres between them. The shape and size of the bond impression approximately corresponds to the shape and size of the bonding surface of a bonding protrusion on the calender roller.

A "column" of bonds on a nonwoven web is a group of nearest neighboring bonds of like shape and rotational orientation that are arranged along the line that extends most predominately in the machine direction.

"Cross direction"(CD) - with respect to the making of a nonwoven web material and the nonwoven web material, refers to the direction along the web material substantially perpendicular to the direction of forward travel of the web material through the manufacturing line in which the web material is manufactured. With respect to a batt moving through the nip of a pair of calender rollers to form a bonded nonwoven web, the cross direction is perpendicular to the direction of movement through the nip, and parallel to the nip.

"Disposable" is used in its ordinary sense to mean an article that is disposed or discarded after a limited number of usage events over varying lengths of time, for example, less than about 20 events, less than about 10 events, less than about 5 events, or less than about 2 events.

"Diaper" refers to an absorbent article generally worn by infants and incontinent persons about the lower torso so as to encircle the waist and legs of the wearer and that is specifically adapted to receive and contain urinary and fecal waste. As used herein, term "diaper" also includes "pant" which is defined below.

"Fiber" and "filament" are used interchangeably.

"Fiber diameter" is expressed in units of $\mu$m. The terms "grams of fiber per 9000 m" (denier or den) or "grams of fiber per 10000 m" (dTex) are used to describe the fineness or coarseness of fibers, which are linked to the diameter (when assumed to be circular) by the density of the employed material(s).

"Film" - means a skin-like or membrane-like layer of material formed of one or more polymers, which does not have a form consisting predominately of a web-like structure of consolidated polymer fibers and/or other fibers.

"Length" or a form thereof, with respect to a diaper or training pant, refers to a dimension measured along a direction perpendicular to the waist edges and/or parallel to the longitudinal axis.

"Machine direction" (MD) - with respect to the making of a nonwoven web material and the nonwoven web material, refers to the direction along the web material substantially parallel to the direction of forward travel of the web material through the manufacturing line in which the web material is manufactured. With respect to a nonwoven batt moving through the nip of a pair of calender rollers to form a bonded nonwoven web, the machine direction is parallel to the direction of movement through the nip, and perpendicular to the nip.

"Monocomponent" refers to fiber formed of a single polymer component or single blend of polymer components, as distinguished from bicomponent or multicomponent fiber.

"Multicomponent" refers to fiber having a cross-section comprising more than one discrete polymer component, more than one discrete blend of polymer components, or at least one discrete polymer component and at least one discrete blend of polymer components. "Multicomponent fiber" includes, but is not limited to, "bicomponent fiber." A multicomponent fiber may have an overall cross section divided into subsections of the differing components of any shape or arrangement, including, for example, coaxial subsections, core-and-sheath subsections, side-by-side subsections, radial subsections, islands-in-the-sea, etc.

A "nonwoven" is a manufactured sheet or web of directionally or randomly oriented fibers which are first formed into a batt and then consolidated and bonded together by friction, cohesion, adhesion or one or more patterns of bonds and bond impressions created through localized compression and/or application of pressure, heat, ultrasonic or heating energy, or a combination thereof. The term does not include fabrics which are woven, knitted, or stitch-bonded with yarns or filaments. The fibers may be of natural or man-made origin and may be staple or continuous filaments or be formed in situ. Commercially available fibers have diameters ranging from less than about 0.001 mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). Nonwoven fabrics can be formed by many processes including but not limited to meltblowing, spunbonding, spunmelting, solvent spinning, electrospinning, carding, film fibrillation, melt-film fibrillation, airlaying, dry-laying, wetlaying with staple fibers, and combinations of these processes as known in the art. The basis weight of nonwoven fabrics is usually expressed in grams per square meter (gsm).

"Opacity" is a numeric value relating to the ability of a web material to transmit light therethrough, measured according the Opacity Measurement Method set forth herein.

"Pant" or "training pant", as used herein, refer to disposable garments having a waist opening and leg openings designed for infant or adult wearers. A pant may be placed in position on the wearer by inserting the wearer's legs into the leg openings and sliding the pant into position about a wearer's lower torso. A pant may be preformed by any suitable technique including, but not limited to, joining together portions of the article using refastenable and/or non-refastenable bonds (*e.g.*, seam, weld, adhesive, cohesive bond, fastener, etc.). A pant may be preformed anywhere along the circumference of the article (*e.g.,* side fastened, front waist fastened). While the terms "pant" or "pants" are used herein, pants are also commonly referred to as "closed diapers," "prefastened diapers," "pull-on diapers," "training pants," and "diaper-pants". Suitable pants are disclosed in U.S. Pat. No. 5,246,433, issued to Hasse, et al. on September 21, 1993; U.S. Pat. No. 5,569,234, issued to Buell et al. on October 29, 1996; U.S. Pat. No. 6,120,487, issued to Ashton on September 19, 2000; U.S. Pat. No. 6,120,489, issued to Johnson et al. on September 19, 2000; U.S. Pat. No. 4,940,464, issued to Van Gompel et al. on July 10, 1990; U.S. Pat. No. 5,092,861, issued to Nomura et al. on March 3, 1992; U.S. Patent Publication No. 2003/0233082 A1, entitled "Highly Flexible And Low Deformation Fastening Device", filed on June 13, 2002; U.S. Pat. No. 5,897,545, issued to Kline et al. on April 27, 1999; U.S. Pat. No. 5,957,908, issued to Kline et al on September 28, 1999.

When used as an adjective in connection with a component of a material, the term "predominately" means that the component makes up greater than 50% by weight of the material. When used as an adjective in connection with a directional orientation of a physical feature or geometric attribute thereof, "predominately" means the feature or attribute has a projection onto a line extending along the direction indicated, greater in length than the projection onto a line perpendicular thereto. Within other context, the term "predominantly" refers to a condition which imparts a substantial effect on a property or feature. Thus, when a material comprises "predominantly" a component said to impart a property, this component imparts a property that the material otherwise would not exhibit. For example, if a material comprises "predominantly" heat-fusible fibers, the quantity and components of these fibers must be sufficient to allow heat fusion of the fibers.

A "bonding protrusion" or "protrusion" is a feature of a bonding roller at its radially outermost portion, surrounded by recessed areas. Relative the rotational axis of the bonding roller, a bonding protrusion has a radially outermost bonding surface with a bonding surface shape and a bonding surface shape area, which generally lies along an outer cylindrical surface with a substantially constant radius from the bonding roller rotational axis; however, protrusions having bonding surfaces of discrete and separate shapes are often small enough relative the radius of the bonding roller that the bonding surface may appear flat/planar; and the bonding surface shape area is closely approximated by a planar area of the same shape. A bonding protrusion may have sides that are perpendicular to the bonding surface, although usually the sides have an angled slope, such that the cross section of the base of a bonding protrusion is larger than its bonding surface. A plurality of bonding protrusions may be arranged on a calender roller in a pattern. The plurality of bonding protrusions has a bonding area per unit surface area of the outer cylindrical surface which can be expressed as a percentage, and is the ratio of the combined total of the bonding shape areas of the protrusions within the unit, to the total surface area of the unit.

A "row" of bonds on a nonwoven web is a group of nearest neighboring bonds of like shape and rotational orientation that are arranged along the line that extends most predominately in the cross direction.

"Tensile Strength" refers to the maximum tensile force (Peak Force) a material will sustain before tensile failure, as measured by the Tensile Strength Measurement Method set forth herein.

"Thickness" and "caliper" are used herein interchangeably.

"Total Stiffness" refers to the measured and calculated value relating to a material, according to the Stiffness measurement method set forth herein.

"Width" or a form thereof, with respect to a diaper or training pant, refers to a dimension measured along a direction parallel to the waist edges and/or perpendicular to the longitudinal axis.

"Z-direction," with respect to a web, means generally orthogonal or perpendicular to the plane approximated by the web in the machine and cross direction dimensions.

Absorbent Article

Examples of the present invention include disposable absorbent articles having improved softness attributes.

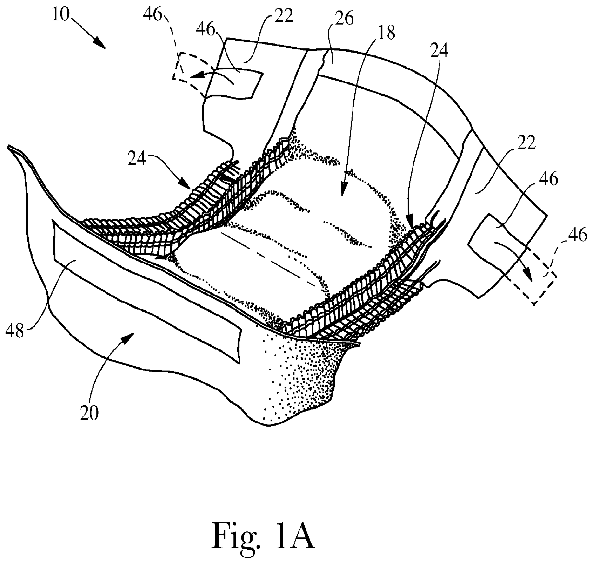

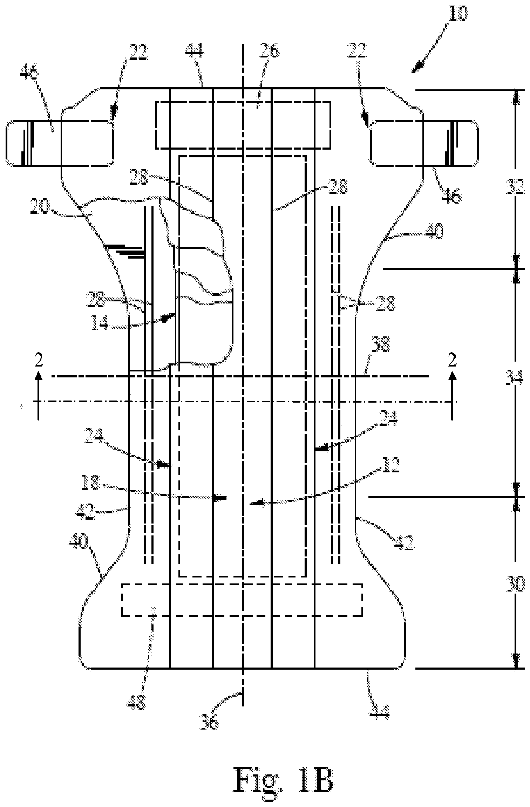

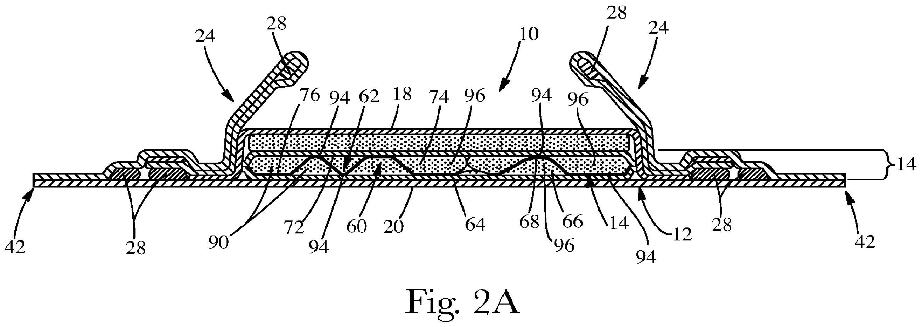

Fig. 1A is a perspective view of a diaper 10 in a relaxed, laid-open position as it might appear opened and lying on a horizontal surface. Fig. 1B is a plan view of a diaper 10 shown in a flat-out, uncontracted state (*i.e.*, without elastic induced contraction), shown with portions of the diaper 10 cut away to show underlying structure. The diaper 10 is depicted in Fig. 1B with its longitudinal axis 36 and its lateral axis 38. Portions of the diaper 10 that contact a wearer are shown oriented upwards in Fig. 1A, and are shown facing the viewer in Fig. 1B. Fig. 2A is a cross section of the diaper taken at line 2-2 in Fig. 1B.

The diaper 10 generally may comprise a chassis 12 and an absorbent core 14 disposed in the chassis. The chassis 12 may comprise the main body of the diaper 10.

The chassis 12 may include a topsheet 18, which may be liquid pervious, and a backsheet 20, which may be liquid impervious. The absorbent core 14 may be encased between the topsheet 18 and the backsheet 20. The chassis 12 may also include side panels 22, elasticized leg cuffs 24, and an elastic waist feature 26. The chassis 12 may also comprise a fastening system, which may include at least one fastening member 46 and at least one landing zone 48.

The leg cuffs 24 and the elastic waist feature 26 may each typically comprise elastic members 28. One end portion of the diaper 10 may be configured as a first waist region 30 of the diaper 10. An opposite end portion of the diaper 10 may be configured as a second waist region 32 of the diaper 10. An intermediate portion of the diaper 10 may be configured as a crotch region 34, which extends longitudinally between the first and second waist regions 30 and 32. The crotch region 34 may include from 33.3% to 50% of the overall length of the diaper 10, and each of waist regions 30, 32 may correspondingly include from 25% to 33.3% of the overall length of the diaper 10.

The waist regions 30 and 32 may include elastic elements such that they gather about the waist of the wearer to provide improved fit and containment (elastic waist feature 26). The crotch region 34 is that portion of the diaper 10 which, when the diaper 10 is worn, is generally positioned between the wearer's legs.

The diaper 10 may also include such other features including front and rear ear panels, waist cap features, elastics and the like to provide better fit, containment and aesthetic characteristics. Such additional features are described in, *e.g.,* U.S. Pats. Nos. 3,860,003 and 5,151,092.

In order to apply and keep diaper 10 in place about a wearer, the second waist region 32 may be attached by the fastening member 46 to the first waist region 30 to form leg opening(s) and an article waist. When fastened, the fastening system carries a tensile load around the article waist.

According to some examples, the diaper 10 may be provided with a re-closable fastening system or may alternatively be provided in the form of a pant-type diaper. When the absorbent article is a diaper, it may comprise a re-closable fastening system joined to the chassis for securing the diaper to a wearer. When the absorbent article is a pant-type diaper, the article may comprise at least two side panels joined to the chassis and to each other to form a pant. The fastening system and any component thereof may include any material suitable for such a use, including but not limited to plastics, films, foams, nonwoven, woven, paper, laminates, stretch laminates, activated stretch laminates, fiber reinforced plastics and the like, or combinations thereof. In some examples, the materials making up the fastening device may be flexible. In some examples, the fastening device may comprise cotton or cotton-like materials for additional softness or consumer perception of softness. The flexibility may allow the fastening system to conform to the shape of the body and thus, reduce the likelihood that the fastening system will irritate or injure the wearer's skin.

For unitary absorbent articles, the chassis 12 and absorbent core 14 may form the main structure of the diaper 10 with other features added to form the composite diaper structure. While the topsheet 18, the backsheet 20, and the absorbent core 14 may be assembled in a variety of well-known configurations, preferred diaper configurations are described generally in U.S. Pat. No. 5,554,145 entitled "Absorbent Article With Multiple Zone Structural Elastic-Like Film Web Extensible Waist Feature" issued to Roe et al. on Sep. 10, 1996; U.S. Pat. No. 5,569,234 entitled "Disposable Pull-On Pant" issued to Buell et al. on Oct. 29, 1996; and U.S. Pat. No. 6,004,306 entitled "Absorbent Article With Multi-Directional Extensible Side Panels" issued to Robles et al. on Dec. 21, 1999.

The topsheet 18 may be fully or partially elasticized and/or may be foreshortened to create a void space between the topsheet 18 and the absorbent core 14. Exemplary structures including elasticized or foreshortened topsheets are described in more detail in U.S. Pat. No. 5,037,416 entitled "Disposable Absorbent Article Having Elastically Extensible Topsheet" issued to Allen et al. on Aug. 6, 1991; and U.S. Pat. No. 5,269,775 entitled "Trisection Topsheets for Disposable Absorbent Articles and Disposable Absorbent Articles Having Such Trisection Topsheets" issued to Freeland et al. on Dec. 14, 1993.

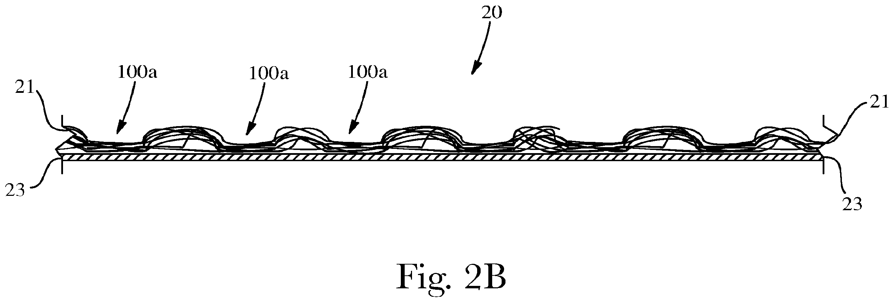

The backsheet 20 may be joined with the topsheet 18. The backsheet 20 may serve prevent the exudates absorbed by the absorbent core 14 and contained within the diaper 10 from soiling other external articles that may contact the diaper 10, such as bed sheets and clothing. Referring to Fig. 2B, the backsheet 20 may be substantially impervious to liquids (*e.g.*, urine) and comprise a laminate of a nonwoven 21 and a thin polymeric film 23 such as a thermoplastic film having a thickness of about 0.012 mm (0.5 mil) to about 0.051 mm (2.0 mils). Suitable backsheet films include those manufactured by Tredegar Industries Inc. of Terre Haute, Ind. and sold under the trade names X15306, X10962, and X10964. Other suitable backsheet materials may include breathable materials that permit vapors to escape from the diaper 10 while still preventing liquid exudates from passing through the backsheet 20. Exemplary breathable materials may include materials such as woven webs, nonwoven webs, composite materials such as film-coated nonwoven webs, and microporous films such as manufactured by Mitsui Toatsu Co., of Japan under the designation ESPOIR and by EXXON Chemical Co., of Bay City, Texas, under the designation EXXAIRE. Suitable breathable composite materials comprising polymer blends are available from Clopay Corporation, Cincinnati, Ohio under the name HYTREL blend PI 8-3097. Other examples of such breathable composite materials are described in greater detail in PCT Application No. WO 95/16746, published on Jun. 22, 1995 in the name of E. I. DuPont. Other breathable backsheets including nonwoven webs and apertured formed films are described in U.S. Pat. No. 5,571,096 issued to Dobrin et al. on Nov. 5, 1996.

In some examples, the backsheet may have a water vapor transmission rate (WVTR) of greater than about 2,000 g/24h/m2, greater than about 3,000 g/24h/m2, greater than about 5,000 g/24h/m2, greater than about 6,000 g/24h/m2, greater than about 7,000 g/24h/m2, greater than about 8,000 g/24h/m2, greater than about 9,000 g/24h/m2, greater than about 10,000 g/24h/m2, greater than about 11,000 g/24h/m2, greater than about 12,000 g/24h/m2, greater than about 15,000 g/24h/m2, measured according to WSP 70.5 (08) at 37.8 0C and 60% Relative Humidity.

Various components of an absorbent article such as a diaper 10 may be formed of a nonwoven web material described herein, including but not necessarily limited to the topsheet 18 and the nonwoven 21 component of the backsheet 20.

Soft Blend Resin

The nonwoven 21 may be formed from one or more resins of polyolefins, polyesters, polyamide including but not limited to polypropylene (PP), polyethylene (PE), and polyethylene terephthalate (PET), poly-lactic acid (PLA), and blends thereof. Resins including polypropylene may be particularly useful because of polypropylene's relatively low cost and surface friction properties of fibers formed from it (*i.e.*, they have a relatively smooth, slippery tactile feel). Resins including polyethylene may also be desirable because of polyethylene's relative softness/pliability and even more smooth/slippery surface friction properties. Relative each other, PP currently has a lower cost and fibers formed from it have a greater tensile strength, while PE currently has a greater cost and fibers formed from it have a lower tensile strength but greater pliability and a more smooth/slippery feel. Accordingly, it may be desirable to form nonwoven web fibers from a blend of PP and PE resins, finding a balance of the best proportions of the polymers to balance their advantages and disadvantages. In some examples, the fibers may be formed of PP/PE blends such as described in U.S. Pat. No. 5,266,392. Nonwoven fibers may be formed of, or may include as additives or modifiers, components such as aliphatic polyesters, thermoplastic polysaccharides, or other biopolymers.

In one embodiment, the nonwoven 21 comprises at least a layer of fibers that are made of a composition comprising a first polyolefin, a second polyolefin that is different than the first polyolefin and a softness enhancer additive. In one embodiment, the first polyolefin may be polypropylene homopolymer. It is found that a second polyolefin comprising a propylene copolymer can provide advantageous properties to the resulting nonwoven. A "propylene copolymer" includes at least two different types of monomer units, one of which is propylene. Suitable examples of monomer units include ethylene and higher alpha-olefins ranging from $C_4$ - $C_{20}$, such as, for example, 1-butene, 4-methyl-1-pentene, 1-hexene or 1-octene and 1-decene, or mixtures thereof, for example. Preferably, ethylene is copolymerized with propylene, so that the propylene copolymer includes propylene units (units on the polymer chain derived from propylene monomers) and ethylene units (units on the polymer chain derived from ethylene monomers).

Typically the units, or comonomers, derived from at least one of ethylene or a C4-10 alpha-olefin may be present in an amount of 1% to 35%, or 5% to about 35%, or 7% to 32%, or 8 to about 25%, or 8% to 20%, or even 8% to 18% by weight of the propylene-alpha-olefin copolymer. The comonomer content may be adjusted so that the propylene-alpha-olefin copolymer has preferably a heat of fusion ("DSC") of 75 J/g or less, melting point of 100 °C or less, and crystallinity of 2% to about 65% of isotactic polypropylene, and preferably a melt flow rate (MFR) of 0.5 to 90 dg/min.

In one embodiment, the propylene-alpha-olefin copolymer comprises of ethylene-derived units. The propylene-alpha-olefin copolymer may contain 5% to 35%, or 5% to 20%, or 10% to 12%, or 15% to 20%, of ethylene-derived units by weight of the propylene-alpha-olefin copolymer. In some embodiments, the propylene-alpha-olefin copolymer consists essentially of units derived from propylene and ethylene, *i.e.*, the propylene-alpha-olefin copolymer does not contain any other comonomer in an amount typically present as impurities in the ethylene and/or propylene feedstreams used during polymerization or an amount that would materially affect the heat of fusion, melting point, crystallinity, or melt flow rate of the propylene-alpha-olefin copolymer, or any other comonomer intentionally added to the polymerization process.

The propylene-alpha-olefin copolymer may have a triad tacticity of three propylene units, as measured by 13C NMR, of at least 75%, at least 80%, at least 82%, at least 85%, or at least 90%. The "Triad tacticity" is determined as follows. The tacticity index, expressed herein as "m/r", is determined by 13C nuclear magnetic resonance ("NMR"). The tacticity index m/r is calculated as defined by H. N. Cheng in 17 MACROMOLECULES 1950 (1984), incorporated herein by reference. The designation "m" or "r" describes the stereochemistry of pairs of contiguous propylene groups, with "m" referring to meso and "r" referring to racemic. An m/r ratio of 1.0 generally describes a syndiotactic polymer, and an m/r ratio of 2.0 generally describes an atactic material. An isotactic material theoretically may have a m/r ratio approaching infinity, and many by-product atactic polymer have sufficient isotactic content to result in an m/r ratio of greater than 50.

The propylene-alpha-olefin copolymer may have a heat of fusion ("Hf"), as determined by Differential Scanning calorimetry ("DSC"), of 75 J/g or less, 70 J/g or less, 50 J/g or less, or even 35 J/g or less. The propylene-alpha-olefin copolymer may have a Hf of at least 0.5 J/g, 1 J/g, or at least 5 J/g. The "DSC" is determined as follows. About 0.5 grams of polymer is weighed and pressed to a thickness of about 15 to 20 mils (about 381-508 microns) at about 140-150°C, using a "DSC mold" and MYLAR(TM) film as a backing sheet. The pressed polymer sample is allowed to cool to ambient temperatures by hanging in air (the MYLAR(TM) film backing sheet is not removed). The pressed polymer sample is then annealed at room temperature (about 23-25°C.) for 8 days. At the end of this period, a 15-20 mg disc is removed from the pressed polymer sample using a punch die and is placed in a 10 microliter aluminum sample pan. The disc sample is then placed in a DSC (Perkin Elmer Pyris 1 Thermal Analysis System) and is cooled to -100°C. The sample is heated at about 10°C/min to attain a final temperature of 165°C. The thermal output, recorded as the area under the melting peak of the disc sample, is a measure of the heat of fusion and can be expressed in Joules per gram (J/g) of polymer and is automatically calculated by the Perkin Elmer system. Under these conditions, the melting profile shows two maxima, the maximum at the highest temperature is taken as the melting point within the range of melting of the disc sample relative to a baseline measurement for the increasing heat capacity of the polymer as a function of temperature.

The propylene-alpha-olefin copolymer may have a single peak melting transition as determined by DSC. In one embodiment, the copolymer has a primary peak transition of 90° C. or less, with a broad end-of-melt transition of about 110° C. or greater. The peak "melting point" ("Tm") is defined as the temperature of the greatest heat absorption within the melting range of the sample. However, the copolymer may show secondary melting peaks adjacent to the principal peak, and/or at the end-of-melt transition. For the purposes of this disclosure, such secondary melting peaks are considered together as a single melting point, with the highest of these peaks being considered the Tm of the propylene-alpha-olefin copolymer. The propylene-alpha-olefin copolymer may have a Tm of 100 °C or less, 90 °C or less, 80 °C or less, or 70 °C or less. The propylene-alpha-olefin copolymer may have a density of 0.850 to 0.920 g/cm3, 0.860 to 0.900 g/cm3, or 0.860 to 0.890 g/cm3, at room temperature as measured per ASTM D-1505.

The propylene-alpha-olefin copolymer may have a melt flow rate ("MFR"), as measured according to ASTM D1238, 2.16 kg at 230° C., of at least 0.2 dg/min. In one embodiment, the propylene-alpha-olefin copolymer MFR is 0.5 to 5000 dg/min, about 1 to 2500 dg/min, about 1.5 to 1500 dg/min, 2 to 1000 dg/min, 5 to 500 dg/min, 10 to 250 dg/min, 10 to 100 dg/min, 2 to 40 dg/min, or 2 to 30 dg/min.

The propylene-alpha-olefin copolymer may have an Elongation at Break of less than 2000%, less than 1000%, or less than 800%, as measured per ASTM D412.

The propylene-alpha-olefin copolymer may have a weight average molecular weight (Mw) of 5,000 to 5,000,000 g/mole, preferably 10,000 to 1,000,000 g/mole, and more preferably 50,000 to 400,000 g/mole; a number average molecular weight (Mn) of 2,500 to 2,500,00 g/mole, preferably 10,000 to 250,000 g/mole, and more preferably 25,000 to 200,000 g/mole; and/or a z-average molecular weight (Mz) of 10,000 to 7,000,000 g/mole, preferably 80,000 to 700,000 g/mole, and more preferably 100,000 to 500,000 g/mole. The propylene-alpha-olefin copolymer may have a molecular weight distribution ("MWD") of 1.5 to 20, or 1.5 to 15, preferably 1.5 to 5, and more preferably 1.8 to 5, and most preferably 1.8 to 3 or 4. The "Molecular weight (Mn, Mw, and Mz)" and "MWD" can be determined as follows and as described in Verstate et al., 21 MACROMOLECULES 3360 (1988). Conditions described herein govern over published test conditions. Molecular weight and MWD are measured using a Waters 150 gel permeation chromatograph equipped with a Chromatix KMX-6 on-line light scattering photometer. The system is used at 135[deg.] C. with 1,2,4-trichlorobenze as the mobile phase. Showdex (Showa-Denko America, Inc.) polystyrene gel columns 802, 803, 804, and 805 are used. This technique is discussed in Verstate et al., 21 MACROMOLECULES 3360 (1988). No corrections for column spreading are employed; however, data on generally acceptable standards, *e.g*., National Bureau of Standards Polyethylene 1484, and anionically produced hydrogenated polyisoprenes (an alternating ethylenepropylene copolymer) demonstrate that such corrections on Mw/Mn or Mz/Mw are less than 0.05 units. Mw/Mn was calculated from an elution time-molecular relationship whereas Mz/Mw was evaluated using the light scattering photometer. The numerical analysis can be performed using the commercially available computer software GPC2, MOLWT2 available from LDC/Milton Roy-Rivera Beach, Fla. Examples suitable propylene-alpha-olefin copolymers are available commercially under the trade names VISTAMAXX® (Exxon-Mobil Chemical Company, Houston, Tex., USA), VERSIFY® (The Dow Chemical Company, Midland, Mich., USA), certain grades of TAFMER® XM or NOTIO® (Mitsui Company, Japan), and certain grades of SOFTEL® (Basell Polyolefins of the Netherlands). The particular grade(s) of commercially available propylene-alpha-olefin copolymer suitable for use in the invention can be readily determined using methods applying the selection criteria in the above.

Propylene copolymers have a good mixability with propylene homopolymers, where depending on the mutual ratio of both constituents it is possible to prepare a material exhibiting various properties. A propylene copolymer is soft to touch and the nonwoven textile produced from it has good drapeability and is easy to bend. On the other hand polypropylene provides strength and reduces the plasticity of the material. Examples of composition that are suitable for the manufacturing of fibrous nonwoven materials can include at least 60%, at least 70%, at least 75%, or at least 80% by weight of the composition of polypropylene homopolymer, and at least 10%, at least 12%, at least 14% by weight of the propylene copolymer. The described composition is generally drapable and soft but also maintains the required mechanical properties. However it is found that it can feels rough to the touch and can be described as "rubbery." In particular, propylene-alpha-olefin copolymers, particularly propylene-ethylene copolymers, can be tackier than conventional fibers made from polyolefins such as polyethylene and polypropylene.

Softness enhancer additive

It is found that the addition of a softness enhancer additive can be advantageous to reduce the tacky or rubbery feel of fibers that are made of a composition that includes a blend of the first and second polyolefin previously described. The softness enhancer additive generally imparts a relatively silky (reduced friction) feel to the fibers. The softness enhancer additive may be added to the composition in neat form, diluted, and/or as a masterbatch in, for example, polyolefin polymers such as polypropylene, polystyrene, low density polyethylene, high density polyethylene, or propylene-alpha-olefin copolymers.

A composition suitable to make fibers as described herein contains one or more softness enhancer additive, which can be present in an amount of between 0.01% to 10%, or between 0.03% to 5%, or even between 0.05% to 1% by weight of the fibers. Once the fiber are spun to form a nonwoven, some of the softness enhancer additive may volatilize and no longer be present in the same amount in the fibers forming the nonwoven, It is also believed that some of the softness enhancer additive may migrate from the interior portion of the fiber to the outer surface of the fiber. Without intending to be bound by any theory, it is believed that this migration of the additive to the outer surface of the fiber may contribute to the perception of softness that a user experiences when she touches the nonwoven material.

In one embodiment, the softness enhancer additive is an organic amine compound, i.e., contains an amine group bound to a hydrocarbon group. In another embodiment, the softness enhancer additive is a fatty acid amine or a fatty acid amide. In some embodiments, the softness enhancer additive may have one or more paraffinic or olefinic groups bound to a nitrogen atom, forming an amine or an amide compound. The paraffinic or olefinic group may be, for example, a polar or ionic moiety as a side chain or within the amine/amide backbone. Such polar or ionic moieties can include hydroxyl groups, carboxylate groups, ether groups, ester groups, sulfonate groups, sulfite groups, nitrate groups, nitrite groups, phosphate groups, and combinations thereof.

In one embodiment, the softness enhancer additive is an alkyl-ether amine having the formula $(R'OH)_{3-x}NR_x$, wherein R is selected from the group consisting of hydrogen, $C_{1-40}$ alkyl radicals, $C_{2-40}$ alkylethers, $C_{1-40}$ alkylcarboxylic acids, and $C_{2-40}$ alkylesters; R' is selected from the group consisting of $C_{1-40}$ alkyl radicals, $C_{2-40}$ alkylethers, $C_{1-40}$ carboxylic acids, and $C_{2-40}$ alkylesters; and x is 0, 1, 2 or 3, preferably 0 or 1, more preferably 1. In one embodiment, R is selected from the group consisting of hydrogen and $C_{5-40}$ alkyl radicals; and R' is selected from the group consisting of $C_{5-40}$ alkyl radicals and $C_{5-40}$ alkylethers.

In another embodiment, the softness enhancer additive is an amide-containing compound having the formula: $RCONH_2$, wherein R is a $C_{5-23}$ alkyl or alkene. In another embodiment, the softness enhancer additive is a fatty acid amide having the formula: $(R'CO)_{3-x}NR''_x$, wherein R" is selected from the group consisting of hydrogen, $C_{10-60}$ alkyl radicals and $C_{10-60}$ alkene radicals and substituted versions thereof; R' is selected from the group consisting of $C_{10-60}$ alkyl radicals, $C_{10-60}$ alkene radicals, and substituted versions thereof; and x is 0, 1, 2 or 3, preferably 1 or 2, more preferably 2. As used herein, an "alkene" radical is a radical having one or more unsaturated double-bonds in the radical chain (*e.g.*,-CH2CH2CH2CH2CH=CHCH2CH2CH2CH.sub-.2CH2CH3), and "substituted" means substitution anywhere along the hydrocarbon chain of a hydroxyl group, carboxyl group, halide, or sulfate group.

In some embodiments, the softness enhancer additive contains an unsaturated amide. In one embodiment, the unsaturated amide-containing softness enhancer additive has the formula: $RCONH_2$, wherein R is a $C_{5-23}$ alkene.

In another embodiment, the unsaturated amide-containing softness enhancer additive has the formula: $(R'CO)3-xNR''x$, wherein R'' is selected from the group consisting of hydrogen, C10-60 alkyl radicals and C10-60 alkene radicals and substituted versions thereof; R' is selected from the group consisting of C10-60 alkene radicals and substituted versions thereof; and x is 0, 1, 2 or 3, preferably 1 or 2, more preferably 2. In some embodiments, the unsaturated amide-containing softness enhancer additive is at least one of palmitoleamide, oleamide, linoleamide, or erucamide. In other embodiments, the unsaturated amide-containing softness enhancer additive is at least one of oleamide or erucamide. In the preferred embodiment the softness enhancer additive contains erucamide.

Non-limiting examples of softness enhancer additives include bis(2-hydroxyethyl) isodecyloxypropylamine, poly(5)oxyethylene isodecyloxypropylamine, bis(2-hydroxyethyl) isotridecyloxypropylamine, poly(5)oxyethylene isotridecyloxypropylamine, bis(2-hydroxyethyl) linear alkyloxypropylamine, bis(2-hydroxyethyl) soya amine, poly(15)oxyethylene soya amine, bis(2-hydroxyethyl) octadecylamine, poly(5)oxyethylene octadecylamine, poly(8)oxyethylene octadecylamine, poly(10)oxyethylene octadecylamine, poly(15)oxyethylene octadecylamine, bis(2-hydroxyethyl) octadecyloxypropylamine, bis(2-hydroxyethyl) tallow amine, poly(5)oxyethylene tallow amine, poly(15)oxyethylene tallow amine, poly(3)oxyethylene-1,3-diaminopropane, bis(2-hydroxyethyl) cocoamine, bis(2-hydroxyethyl)isodecyloxypropylamine, poly(5)oxyethylene isodecyloxypropylamine, bis(2-hydroxyethyl) isotridecyloxypropylamine, poly(5)oxyethylene isotridecyloxypropylamine, bis(2-hydroxyethyl) linear alkyloxypropylamine, bis(2-hydroxyethyl) soya amine, poly(15)oxyethylene soya amine, bis(2-hydroxyethyl) octadecylamine, poly(5)oxyethylene octadecylamine, poly(8)oxyethylene octadecylamine, poly(10)oxyethylene octadecylamine, poly(15)oxyethylene octadecylamine, bis(2-hydroxyethyl) octadecyloxypropylamine, bis(2-hydroxyethyl) tallow amine, poly(5)oxyethylene tallow amine, poly(15)oxyethylene tallow amine, poly(3) oxyethylene-1,3-diaminopropane, bis(2-hydroxethyl) cocoamine, valeramide, caproicamide, erucamide, caprylicamide, pelargonicamide, capricamide, lauricamide, lauramide, myristicamide, myristamide, palmiticamide, palmitoleamide, palmitamide, margaric (daturic) amide, stearicamide, arachidicamide, behenicamide, behenamide, lignocericamide, linoleamide, ceroticamide, carbocericamide, montanicamide, melissicamide, lacceroicamide, ceromelissic (psyllic) amide, geddicamide, 9-octadecenamide, oleamide, stearamide, tallow bis(2-hydroxyethyl)amine, cocobis(2-hydroxyethyl)amine, octadecylbis(2-hydroxyethyl)amine, oleylbis(2-hydroxyethyl)amine, ceroplastic amide, and combinations thereof.

Commercial examples of useful softness enhancer additives include ATMER® compounds (Ciba Specialty Chemicals), ARMID®, ARMOFILM® and ARMOSLIP® compounds and NOURYMIX concentrates (Akzo Nobel Chemicals), CROTAMID® compounds (Croda Universal Inc), CESA SLIP® compounds (Clariant). Further examples of slip additives include compounds from A. Schulman, Germany, Techmer, USA, or Ampacet, USA.

Compositions useful in the invention may include one or more different softness enhancer additives. For example, in one embodiment a composition may contain one or more unsaturated amide-containing softness enhancer additives, and in another embodiment one or more unsaturated amide-containing softness enhancer additives and one or more saturated amide-containing softness enhancer additives. In some embodiments, a composition includes a combination of low molecular weight (Mw) and thus faster migrating amides, *e.g.*, erucamide or oleamide, and higher molecular weight (Mw) and thus slower migrating amides, *e.g.*, behenamide or stearamide. It should be noted, that compounds that are suitable as softness enhancer additives, such as for example amide additives, may sublimate (*i.e.* transform directly from a solid state to a gaseous state) when subjected to high temperatures. One skilled in the art will appreciate that the sublimation level may depend on the additive temperature and partial pressure of additive vapors over the surface exposed to the outside environment. One skilled in the art will also appreciate that the processing temperatures should remain lower than the TGA (*i.e.* Thermogravimetric analysis) Rapid weight loss temperature of the components. Surprisingly it is been found, that when softness enhancer additives of the amide type are added in a spun melting process, it is advantageous to maintain the process temperatures at a level well below the TGA Rapid weight loss temperature. In particular, it is believed that the temperature of the molten composition ahead of the spinnerets should be at least 20°C lower, or even 25°C lower than the TGA Rapid weight loss temperature of the softness enhancer additive. The TGA Rapid weight loss temperature for various substances can be found for example in "Plastics additives: an industrial guide" written by Ernest W.Flick.

Without wishing to be bound by theory it is believed that this sublimation of the additive can be caused by particular process conditions during fiber production. As in typical nonwoven manufacturing processes, the polymer composition is molten and brought to a particular temperature, which enables the composition to flow and be extruded through spinnerets in order to form fibers. The newly formed fibers are then quenched at a much lower temperature by air, which flows against the fibers' outer surface. When the molten composition is heated to a temperature, which causes the softness enhancer additive to overheat, and the additive may evaporate/sublimate from the outer surface of solidifying fiber. Because of the rapid and constant air flow, the partial pressure is kept to a relative low level, which favors evaporation/sublimation of the softness enhancer additive than one would otherwise expect from TGA values. The following table provides temperatures for several amides.

**TGA Weight Loss of Amides**

| Type | Softness Enhancer Additive | % total Weight Loss | Temperature when Weight Loss begins (°C) | Temperature when RapidWeight Loss begins (°C) |
|---|---|---|---|---|
| Primary | Oleamide | 99.3 | 195 | 250 |
| | Erucamide | 94.8 | 220 | 280 |
| Secondary | Oleylpalmitamide | 11.8 | 225 | 300 |
| Bisamide | Ethylenebisoleamide | 11.6 | 220 | 305 |

In some embodiments, in addition to including nonwoven 21 formed of materials as described above, the nonwoven may include two or more layers formed of differing components, as described in, for example, U.S. Pat. App. Ser. Nos. 14/058,376; 14/058,398 and 14/058,479. Calender bonding

Spunbonding includes the step of calender-bonding the batt of spunlaid fibers, to consolidate them and bond them together to some extent to create a fabric-like structure and enhance mechanical properties *e.g*., tensile strength, which may be desirable so the material can sufficiently maintain structural integrity and dimensional stability in subsequent manufacturing processes, and in the final product in use. Calender-bonding may be accomplished by passing the batt through the nip between a pair of rotating calender rollers, thereby compressing and consolidating the fibers in the z-direction to form a web. One or both of the rollers may be heated, so as to promote plastic deformation, intermeshing and/or thermal bonding/fusion between superimposed fibers compressed at the nip. The rollers may form operable components of a bonding mechanism in which they are urged together by a controllable amount of force, so as to exert the desired compressing force/pressure at the nip. In some processes heating may be deemed unnecessary, since compression alone may generate sufficient energy within the fibers to effect bonding, resulting from rapid deformation and frictional heat generated in the fibers as they are urged against each other where they are superimposed, resulting in plastic deformation and intermeshing, and possibly thermal bonding/fusion. In some processes an ultrasonic energy source may be included with the bonding mechanism so as to transmit ultrasonic vibration to the fibers, again, to generate heat energy within them and enhance bonding.

One or both of the bonding rollers may have their circumferential surfaces machined, etched, engraved or otherwise formed to have thereon a pattern of protrusions and recessed areas, so that bonding pressure exerted on the batt at the nip is concentrated at the outward surfaces of the protrusions, and reduced or substantially eliminated at the recessed areas. As a result, an impressed pattern of bonds between fibers forming the web, somewhat corresponding to the pattern of protrusions on the roller, is formed on the nonwoven web. One roller may have a smooth, unpatterned cylindrical surface, and the other may be formed with a pattern as described; this combination will impart a pattern on the web somewhat reflecting the pattern on the formed roller. In some examples both rollers may be formed with patterns, and in particular examples, differing patterns that work in combination to impress a combination pattern on the web such as described in, for example, U.S. Pat. No. 5,370,764.

A repeating pattern of protrusions and recessed areas such as, for example, depicted in Fig. 3A, may be formed onto one roller. The smaller shapes depicted in Fig. 3A represent outlines of rhombus- or diamond-shaped raised surfaces 100 of protrusions, while the areas between them represent recessed areas 101. Each protrusion surface may be imparted with a width $W_{P1}$ (relative the machine direction MD) and a length $L_{P1}$, such that each protrusion surface 100 has an area. Without intending to be bound by theory, it is believed that the visual impact of the bond impressions impressed on the web, as well as the tensile strength, resulting from the protrusion surfaces 100, may be affected by the area of the protrusion surfaces 100. Accordingly, it is believed desirable that the average area of the individual protrusion surfaces 100 be from 0.74 mm$^2$ to 1.12 mm$^2$, or from 0.84 mm$^2$ to 1.02 mm$^2$, or even from 0.88 mm$^2$ to 0.98 mm$^2$. Protrusion surfaces 100 may have diamond shapes as shown, or may have any other suitable shape, although it is believed that a diamond, rectangle, square or oval shape may have the desirable effect of simulating the appearance of stitching, as in a quilt.

As can be seen in Fig. 3A, protrusion surfaces 100 may be arranged such that they substantially circumscribe a repeating pattern of recessed areas 101 in the form of geometric shapes. The geometric shapes may be contiguously arranged as depicted. The geometric shapes may be diamonds or squares, as depicted (and illustrated by dotted outlines 102a, 103a in Fig. 3A), or may have other shapes, including but not limited to triangles, diamonds, parallelograms, other polygons, circles, hearts, moons, etc. In Fig. 3A, it can be seen also that the pattern of geometric shapes repeats in the machine and cross directions at frequencies determined by the dimensions of the shape circumscribed by outline 103a, where outline 103a is drawn through the centers of the protrusion surfaces 100. It can be seen that the dimensions of the shape circumscribed by outline 103a correspond with shape length $L_{S1}$ and shape width $W_{S1}$ as shown in Fig. 3A. (Again, length and width are designated with reference to the machine direction MD.) Without intending to be bound by theory, within the ranges of basis weights of spunbond nonwoven materials contemplated herein, it is believed that the size of the repeating geometrically-shaped recessed areas 101 may be impactful with respect to optimizing both the apparent and actual desired visible "pop" of the pattern.

It may be desired that the shapes circumscribed by the bond impressions repeat at a frequency of from 99 to 149, or from 105 to 143, or even from 111 to 137 per meter, in either or both the machine and cross directions, on the nonwoven web. Referring to Fig. 3B, for example, this would means that length $L_{S2}$ and/or width $W_{S2}$ may each be about 6.7 mm to 10.1 mm, or from 7.0 mm to 9.5 mm, or even from 7.3 mm to 9 mm. Alternatively, it may be desired that the repeating shapes defined by the bond impressions (for example, as illustrated/suggested by the repeating square or diamond shape defined by outline 103a, Fig. 3A), have areas from 52 mm$^2$ to 78 mm$^2$, or from 55 mm$^2$ to 75 mm$^2$, or even from 58 mm$^2$ to 72 mm$^2$.

As noted, calender-bonding may be used to consolidate the spunlaid fiber batt into a fabric-like nonwoven web and to impart mechanical strength, *e.g.*, tensile strength, to the web. Generally, within the ranges contemplated herein, greater percentages of protrusion surface area to total patterned roller surface area on a roller formed with a given pattern impart greater tensile strength to the web, than lesser percentages. However, this may come at the cost of added stiffness in the web, which may negatively impact tactile softness attributes. It is believed that a suitable balance between imparting sufficient tensile strength for subsequent processing and satisfactory structural strength in the finished product, and preserving tactile softness attributes, may be struck when the ratio of area of the protrusion surfaces (*e.g.*, protrusion surfaces 100, Fig. 3A) to the total patterned roller surface area is at least 10%, preferably not more than 20%, more preferably 10% to 17%, and even more preferably 10% to 15%.

It will be noted in Fig. 3A that protrusion surfaces 100 appear to form interrupted paths, in the example depicted, along directions indicated by arrows 104a, 104b. Without intending to be bound by theory, it is believed that the interruptions provide at least two beneficial effects. First, it is believed that the resulting bond impressions in the web have the effect of simulating the appearance of stitching, as in a quilt. Second, it is believed that the interruptions in the bond paths provide a multitude of natural hinge points at which the web may flex about the discrete bonds, helping to preserve or enhance pliability in the web despite the presence of the bonds. When a spunlaid batt is passed through a nip formed by a calendering roller having the pattern depicted in Fig. 3A, bonds among and between the fibers are formed beneath protrusion surfaces 100. If these surfaces were continuous along directions 104a, 104b instead of interrupted as shown, the resulting bonds would also be substantially continuous along those directions. This could cause the resulting nonwoven web to be more stiff and less pliable, undesirably comprising its tactile softness attributes.

It will also be noted in Fig. 3A that the directions 104a, 104b followed by the bonding pattern paths may be diagonal with respect to the machine direction. The bond paths imposed on the resulting web will be similarly diagonal with respect to the machine direction. Without intending to be bound by theory, and relative to the use of increased calender-bonding pressure and/or roller temperature to form more fully developed bonds as described herein, it is believed that these paths, when formed of more fully-developed bonds, comprise diagonal paths or linear zones along which the nonwoven web has relatively higher tensile strength and resistance to elongation. It is believed that an interesting effect may result. When these paths are diagonal relative the machine direction and in a criss-crossing pattern as depicted, a net-like structure may be present within the web. As a result, drawing the nonwoven web under tension in the machine direction (as it would be drawn in downstream manufacturing processes, *e.g.*, laminating the nonwoven with a polymer film to form backsheet material) may have the effect of causing the geometric shapes 101 to slightly protrude or "pop" in the z-direction out of the general plane of the web material surface as it narrows in width (exhibiting Poisson effect behavior), or "necks" slightly, as a result of forces within the material under tension in the machine direction, influenced by the net-like structure of diagonal paths of higher tensile strength formed by the bonds.

It is believed that a pattern of diagonally-oriented bonding paths, as suggested in Figs. 3A and 3B, may be more effective for producing the z-direction "pop" effect described above, than other possible configurations. It is believed, further, that along such diagonally-oriented bonding paths, a greater percentage of bonded material along the paths will have a more dramatic impact than a lesser percentage, because the bonding results in the above-mentioned effect of forming a line along the nonwoven of relatively greater tensile strength and resistance to elongation. Thus, referring to Fig. 3B, it can be seen that a line 105 can be traced a path of bond impressions 100a (line 105 is drawn through the centers of bond impressions 100a, in the depicted example). In order that path 105 exhibits sufficiently greater tensile strength and resistance to elongation than neighboring/parallel lines or paths in the material, it may be desirable that the Bond Length Ratio of a segment along line 105 is between 35% and 99%. However, as noted, it may be desirable not to impart too much stiffness to the web (which compromises a tactile softness attribute). Thus, it may be desirable that the Bond Length Ratio of a bond path be between 35% and 80%, more preferably between 35% and 65%, and even more preferably between 35% and 55%.

It has been learned that more fully-developed bonds and a more highly-defined bond pattern may be more effectively achieved when the protrusion surfaces 100 are polished such that they are relatively smooth, rather than having a rougher, machined surface.

In order to complement the z-direction "pop" effect, in which the material forming unbonded areas 101a protrudes out of the general plane approximated by the web surface, it may be desirable that the pattern of bond impressions 100a (*e.g.,* Fig. 3B) be distinct to the naked eye. In order to achieve this, sufficient force between the calendering rollers should be applied, in combination with sufficient heating temperature. As a result, a visibly distinct pattern of bonds may be achieved, and this pattern will have measurable features. Depending upon bonding pressure and temperature used, the shape and area of the protrusion surfaces 100 will be somewhat reflected in the shape and area of the bond impressions in the nonwoven web. Generally it may be desired that calender bonding pressure and/or roller temperature be adjusted so as to cause the shape and area of protrusion surfaces 100 to be substantially reflected in the shape and area of the bond impressions.

It was previously believed that relatively lighter calender bonding pressures and/or relatively cooler bond roller temperatures were required for calender bonding, to avoid tightly binding down fibers, such that they were no longer available to be fluffed by downstream hydroengorging processes intended to enhance visual and tactile softness attributes. Similarly, while creating more fully developed bonds was thought to be required to improve tensile strength properties, it was believed that creating more fully-developed, rigid bonds through relatively greater calendering pressures and/or roller temperatures would have the effect of undesirably increasing stiffness of the nonwoven, unacceptably compromising its tactile softness attributes. In short, it was believed that to preserve or gain tactile and visual softness signals, it was necessary to compromise tensile strength.

However, it has been discovered, surprisingly, that under the circumstances and conditions described herein, negative effects of greater bonding pressures and/or temperatures upon tactile softness attributes may be insubstantial and/or may be overcome by the positive effects upon visual softness attributes. More particularly, it has been discovered that a more highly-defined bond pattern and quilt "pop" may be enabled through use of relatively increased calender bonding pressures and/or roller temperatures, resulting in more fully-developed bonds, which appears to be effective at creating a product that creates overall visual impressions of softness that may overcome any negative effects upon tactile softness signals resulting from increased stiffness in the material.

Further, it has been discovered, surprisingly, that one tactile softness signal, pliability (sometimes known as "drape"), can be substantially maintained or only insubstantially affected with the nonwoven materials under circumstances contemplated herein, even where more fully developed bonds are created, using bond patterns having features such as those described above. Without intending to be bound by theory, it is believed that interruptions in the bond paths, for example, such as those described above (*e.g.,* interruptions 106, Fig. 3B), may provide natural hinge points at which the material may flex easily about bonds, even in the presence of more fully developed bonds. Although the phenomenon is not thoroughly understood, it is believed that this hinge effect, combined with the multitude of relatively small bond sites separated by unbonded areas such as described and depicted herein (*e.g.,* unbonded areas 101a, Fig. 3A), result in effective substantial preservation of pliability or drape even when the bond sites are more fully developed through relatively increased calender bonding pressures and/or temperatures.

At the same time, creating more fully developed bond sites may add tensile strength in the machine and/or cross directions. Thus, counterintuitively, it has been discovered that tensile strength can be substantially increased through creation of more fully developed calender bonds, without a corresponding, deleteriously substantial negative effect on a tactile softness signal, pliability. This effect may be achievable using features of roller patterns as described herein, with suitably adjusted calender force/pressure and roller temperature, to impress a Bond Area Percentage in the nonwoven web of at least 10%, preferably not more than 20%, more preferably 10% to 17%, and even more preferably 10% to 15%.

Referring to Figs. 3A and 3B by way of example, it is believed that the size, shape and area of bond impressions 100a in the nonwoven web product will somewhat, but not identically, reflect the size, shape and area of calender roller protrusion surfaces 100. It is believed that the extent to which the area(s) of bond impressions 100a reflect the area(s) of roller protrusion surfaces 100 may be affected by the bonding force/pressure between the calender rollers at the nip, and/or the roller temperature, and generally, that increasing bonding force/pressure and/or roller temperature will increase the area of bond impressions 100a relative the area of protrusion surfaces 100. Thus, if the area of a protrusion surface 100 is from $0.74 \text{ mm}^2$ to $1.12 \text{ mm}^2$, or from $0.84 \text{ mm}^2$ to $1.02 \text{ mm}^2$, or even from $0.88 \text{ mm}^2$ to $.098 \text{ mm}^2$ as set forth above, it is believed that generally the area of a corresponding bond impression will be somewhat less. In order to achieve the visibly improved results realized in Example 2 herein, bond impressions 100a were created having an average surface area of $0.57 \pm 0.06 \text{ mm}^2$, resulting from protrusion surfaces 100 having an average surface area of approximately $0.93 \text{ mm}^2$. In a prior version, using the same basis weight spunbond batt and the same rollers, the resulting average bond impression surface area was measured as $0.27 \pm 0.02 \text{ mm}^2$, resulting from a relatively lighter calender pressure and/or roller temperature. It is believed that an increase in calender pressure and/or roller temperature is at least partially the cause for the difference.

In other embodiments, the web 21 may be bonded using the techniques and patters described in, for example, U.S. Pat. App. Pub. No. 2013/0253461.

Hydroengorging

Following calender bonding, the web may be subjected to a hydroengorgement process such as described in U.S. Pat. App. Pub. No. 2006/0057921. A distinguishing feature of hydroengorgement, as compared with traditional hydroentanglement, is the use of hydraulic jets to enhance the loft and softness attributes of a nonwoven. However, prior use of hydroengorgement has not been fully satisfactory for providing a nonwoven having both improved softness and a bond pattern with a visually distinct appearance. The '921 application describes a hydroengorgement process involving particular ranges of pressure, *e.g.*, 180-240 bar (2,610-3,480 p.s.i.) applied to the water jet orifices, which was believed required to obtain a desired amount of fluffing of the nonwoven fibers, adding apparent and actual loft or caliper. However, it has been discovered that substantially reducing the hydroengorgement pressure from these magnitudes may still provide desired fluffing without deleterious effects. It is believed that hydroengorgement pressures of the magnitude specified in the '921 application may result in a loss of distinctiveness and/or obscuring of the pattern of bond impressions. Substantially reducing hydroengorgement pressure and energy, and directing hydroengorgement jets at only the inner-facing surface of the nonwoven (thus urging fibers/portions thereof impinged by water jets toward the outer-facing surface) appears to have had a contribution to improving the definition and visual "pop" of the quilt appearance on the outer-facing surface imparted by the roller pattern. A reduced pressure of about 25-100 bars (360-1,450 p.s.i.) may be employed for hydraulic treatment. More preferably, two injectors, each at about 50 bars (725 p.s.i.) of pressure are used, providing an energy transmission of about 0.02 kwhr/kg. It is believed that the use of a one-sided hydroengorgement significantly improves the softness attributes of the nonwoven while pushing fibers in between bond areas to create a more pronounced appearance. Further, the hydroengorgement may enhance the resilience of the pattern such that it can maintain a pronounced appearance after being processed into an article and packaged.

Test Methods

In-Bag Compression Measurement Test

I. DETERMINE FREE STACK HEIGHT

Equipment

• Universal Diaper Packaging Tester (UDPT), including a vertical sliding plate for adding weights. It is counter-balanced by a suspended weight to assure that no downward force is added from the vertical sliding plate assembly to the diaper package at all times. The UDPT is available from Matsushita Industry Co. LTD, 7-21-101, Midorigaoka-cho, Ashiya-city, Hyogo JAPAN, Zip code: 659-0014. For further details concerning this Tester, *see* U.S. Pat. App. Pub. No. 2008/0312624.

• A 850g ($\pm$ 5g) weight.

• Stopwatch with an accuracy to 1 second.

Test Procedure

A) Apparatus Calibration

• Pull down the vertical sliding plate until its bottom touches the tester base plate.

• Set the digital meter located at the side of the vertical sliding scale to zero mark.

• Raise the vertical sliding plate away from the tester base plate.

B) Definitions

• Before-bagger free height refers to the free height data measured on 10 pads of fresh diapers.

- Fresh Diapers - 10 diapers that have never been compressed (stack should be removed (where safely possible) immediately after exit from stacker, before any compression has occurred. If this is not possible, they should be removed from the fingers of a safely stopped stacker chain).

- Out-bag free height designates the free height data measured on 10 pads of aged diapers.

- Aged Diapers - 10 diapers that have been held under compression for approximately 1 minute and/or longer (*i.e.* 10 diapers come from a freshly opened diaper package).

C) Free Height Measurement

- Select 10 adjacent pads of diapers out of the middle from an appropriate source; Fresh diapers for before-bagger free height; Aged diapers for out-of-bag free height.

- Neatly stack these 10 pads of diapers underneath the vertical sliding plate. (Align the center of the top pad directly below the central counter sunk hole of the vertical sliding plate.) • Place the 850 g weight onto the vertical sliding plate.

- Allow the vertical sliding plate to slide down until its bottom lightly touches desired highest point of the stack.

- Measure the stack dimensions in mm by reading the value that appears on the digital meter.

- Remove the weight.

- Raise the vertical sliding plate away from the stack and remove the stack.

- Record the stack height reading to the nearest 1 mm shown on the digital meter.

Procedure -Aging Profile

A) Collect a minimum of three data points from different sample sets *e.g.*, Measure first point from fresh diapers, *e.g.*, measure second point from diapers being aged in bag for 30 mm / 1 hr/ 6 hr/ 12 hr /24 hr, *e.g.*, measure third point from diapers being aged in bag for 5 days or longer.

B) Repeat the three steps as described in "Test Procedure" steps A), C), and D).

Procedure - Out-of-Bag Free Height Recovery

A) Collect 10 pads of fresh/aged diapers.

B) Repeat the first two steps as described in "Test Procedure" steps A) and C).

C) Repeat the steps above for general free height measurement except changing the waiting time (*i.e.*, measure first point at 1 min and remaining points at30 mm/1 hr/6hr/12 hr/I day/3days/5 days, or longer).

Calculation/Reporting

- Report Sample Identification, *i.e.* complete description of product being tested (product brand name/size).

- Report the determined value for all measurement to the nearest 1 mm.
  NOTE: In case of a series of measurements report the number of tested samples, and calculate/report the Average, Standard deviation, Minimum and Maximum of the values.

- Report the Production Date of the measured package (taken from package coding).

- Report the Testing Date and Analytical Method used (GCAS).

II. DETERMINE IN-BAG STACK

Equipment

- Universal Diaper Packaging Tester (UDPT), including a vertical sliding plate for adding weights. It is counter-balanced by a suspended weight to assure that no downward force is added from the vertical sliding plate assembly to the diaper package at all times. The UDPT is available from Matsushita Industry Co. LTD, 7-21-101, Midorigaoka-cho, Ashiya-city, Hyogo JAPAN, Zip code: 659-0014.

- A 850 g ($\pm$ 5 g) weight.

Definitions

- "Package Width" is defined as the maximum distance between the two highest bulging points along the same compression stack axis of a diaper package.

- In-Bag Stack Height = (Package Width I Pad Count Per Stack) x 10 pads of diapers.

Apparatus Calibration

- Pull down the vertical sliding plate until its bottom touches the tester base plate.

- Set the digital meter located at the side of the vertical sliding scale to zero mark.

- Raise the vertical sliding plate away from the tester base plate.

Test Procedure

- Put one of the side panel of the diaper package along its width standing at the center of the tester base plate. Make sure the horizontal sliding plate is pulled to the right so it does not touch the package being tested.

- Add the 850 g weight onto the vertical sliding plate.

- Allow the vertical sliding plate to slide down until its bottom lightly touches desired highest point of the package.

- Measure the package width in mm (distance from the top of the base plate to the top of the diaper package). Record the reading that appears on the digital meter.

- Remove the 850 g weight.

- Raise the vertical sliding plate away from the diaper package.

- Remove the diaper package.

Calculation/Reporting

• Calculate and report the "In-Bag Stack Height" = (Package Width I Pad Count Per Stack) x 10.

• Report Sample Identification, *i.e.* complete description of product being tested (product brand name/size).

• Report the determined value for each measurement (Length/Width/Front-to-Back) to the nearest 1 mm.
NOTE: In case of a series of measurements report the number of tested samples, and calculate/report the Average, Standard deviation, Minimum and Maximum of the values.

• Report the Production Date of the measured package (taken from package coding).

• Report the Testing Date and Analytical Method used (GCAS).

III. CALCULATE %

• Calculate %: 1-(In-Bag Stack Height)/(Free Stack Height) = %

Opacity Measurement Method

The opacity of a material is the degree to which light is blocked by that material. A higher opacity value indicates a higher degree of light block by the material. Opacity may be measured using a 0° illumination / 45° detection, circumferential optical geometry, spectrophotometer with a computer interface such as the HunterLab LabScan XE running Universal Software (available from Hunter Associates Laboratory Inc., Reston, VA). Instrument calibration and measurements are made using the standard white and black calibration plates provided by the vendor. All testing is performed in a room maintained at about 23 $\pm$ 2 °C and about 50 $\pm$ 2 % relative humidity.
Configure the spectrophotometer for the XYZ color scale, D65 illuminant, 10° standard observer, with UV filter set to nominal. Standardize the instrument according to the manufacturer's procedures using the 1.20 inch port size and 1.00 inch area view. After calibration, set the software to the Y opacity procedure.
To obtain the specimen, lay the sample flat on a bench, body facing surface downward, and measure the total longitudinal length of the article. Note a site 33% of the total length from the front waist of the article along the longitudinal axis and a second site, 33% of the total length from the back waist of the article. Carefully remove the backsheet laminate, consisting of both the film and nonwoven web, from the garment-facing side of the article. A cryogenic spray, such as Cyto-Freeze (obtained from Control Company, Houston, TX), may be used to separate the backsheet laminate from the article. Cut a piece 50.8 mm by 50.8 mm centered at each site identified above. Precondition samples at about 23 °C $\pm$ 2 C° and about 50% $\pm$ 2% relative humidity for 2 hours prior to testing.
Place the specimen over the measurement port. The specimen should completely cover the port with the surface corresponding to the garment-facing surface of the article directed toward the port. Cover the specimen with the white standard plate. Take a reading, then remove the white tile and replace it with black standard tile without moving the specimen. Obtain a second reading, and calculate the opacity as follows:

$$\text{Opacity} = \text{Y value}_{(\text{black backing})} \ / \ \text{Y value}_{(\text{white backing})} \ \text{x } 100$$

A total of five identical articles are analyzed and their opacity results recorded. Calculate and report the average opacity and standard deviation for the 10 backsheet laminate measurements to the nearest 0.01%.
Using the same specimens as above, remove the nonwoven web from the film layer for analysis. The cryogenic spray can once again be employed. Precondition samples at about 23 °C $\pm$ 2 C° and about 50% $\pm$ 2% relative humidity for 2 hours prior to testing. In like fashion, analyze the nonwoven web layer following the above procedure. Calculate and report the average opacity and standard deviation for the 10 nonwoven web measurements to the nearest 0.01%.

Average Measured Height Method

Average Measured Height is measured using a GFM Primos Optical Profiler instrument commercially available from GFMesstechnik GmbH, Teltow/Berlin, Germany. The GFM Primos Optical Profiler instrument includes a compact optical measuring sensor based on digital micro-mirror projection, consisting of the following main components: a) DMD projector with 1024x768 direct digital controlled micro-mirrors; b) CCD camera with high resolution (1300x1000 pixels); c) projection optics adapted to a measuring area of at least 27 x 22 mm; d) recording optics adapted to a measuring area of at least 27 x 22 mm; e) a table tripod based on a small hard stone plate; f) a cold light source (an appropriate unit is the KL 1500 LCD, Schott North America, Inc., Southbridge, MA); g) a measuring, control, and evaluation computer running ODSCAD 4.14-1.8 software; and h) calibration plates for lateral (x-y) and vertical (z) calibration available from the vendor.

The GFM Primos Optical Profiler system measures the surface height of a sample using the digital micro-mirror pattern fringe projection technique. The result of the analysis is a map of surface height (z axis) versus displacement in the x-y plane. The system has a field of view of 27 x 22 mm with a resolution of 21 microns. The height resolution should be set to between 0.10 and 1.00 micron. The height range is 64,000 times the resolution. All testing is performed in a conditioned room maintained at about $23 \pm 2$ °C and about $50 \pm 2$ % relative humidity.

To obtain the specimen, lay the sample flat on a bench, body facing surface downward, and measure the total longitudinal length of the article. Note a site 33% of the total length from the front waist of the article along the longitudinal axis and a second site, 33% of the total length from the back waist of the article. Carefully remove the nonwoven outer cover from the garment-facing side of the article. A cryogenic spray, such as Cyto-Freeze (obtained from Control Company, Houston, TX), may be used to separate the nonwoven from the underlying film layer. Cut a piece 40 mm by 40 mm centered at each site identified above. Precondition samples at about 23 °C $\pm$ 2 C° and about 50% $\pm$ 2% relative humidity for 2 hours prior to testing.

Turn on the cold light source. Select settings on the cold light source to give a reading of 3000K on the display (typically 4 and E). Open the ODSCAD 4.14-1.8 Software and select "Start Measurement" and then "Live Pic". Calibrate the instrument according to manufacturer's specifications using the calibration plates for lateral (x-y) and vertical (z) available from the vendor.

Place the 40 mm by 40 mm specimen of nonwoven outer cover, clothing surface upward, under the projection head and on top of a neutral gray surface (WhiBal White Balance Reference, PictureFlow LLC, Melbourne, FL). Ensure that the sample is lying flat, without being stretched. This may be accomplished by taping the perimeter of the sample to the surface or placing it under a weighted frame with an inside dimension of 30 mm x 30 mm.

Using the "Pattern" command, project the focusing pattern on the surface of the specimen. Position the projection head to be normal to the sample surface. Align the projected cross hair with the cross hair displayed in the software. Focus the image using the projector head height adjustment knob. Adjust image brightness according to the instrument manufacturer's instruction by setting the "Projection" value to 10, and then changing the aperture on the lens through the hole in the side of the projector head. Optimum illumination is achieved when the lighting display indicator in the software changes from red to green. Due to variations in instrument configurations, different brightness parameters may be available. Always follow the instrument manufacturer's recommended procedures for proper illumination optimization.

Select the Technical Surface/Standard measurement type. Operating parameters are as follows: Utilization of fast picture recording with a 3 frame delay. A two level Phasecode, with the first level being defined as an 8 pixel strip width with a picture count number of 24, and the second level being defined as a 32 pixel strip width with a picture count number of 6. A full Graycode starting with pixel 1 and ending with pixel 1024. A Prefiltering routine including the removal of invalid pixels, a 5 by 5 median filter, and a 3 by 3 average filter.

Select "Measure" to capture and digitalize the image. The specimen must remain still during this procedure to avoid blurring of the captured image. The image will be captured in approximately 20 seconds. Save the height image and camera image files.

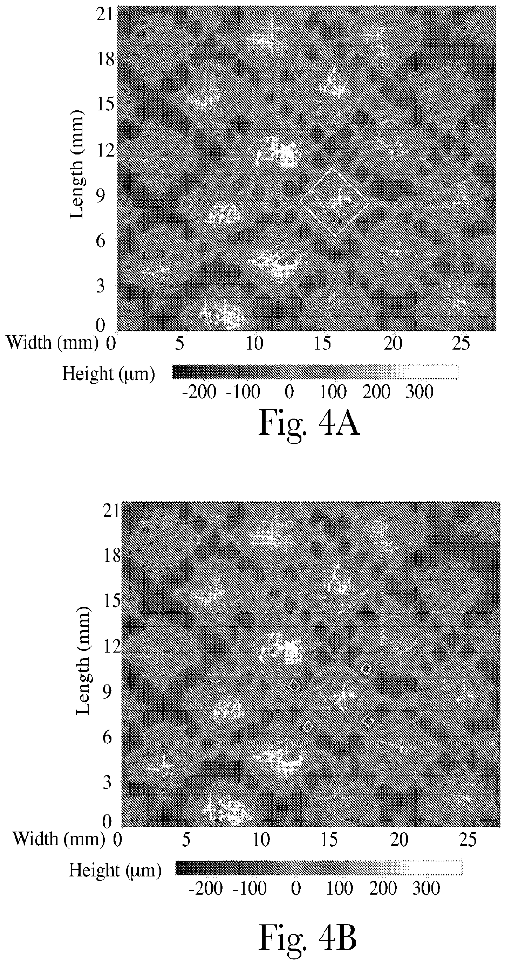

Load the height image into the analysis portion of the software via the clipboard. Zoom in on a region of interest (ROI) encompassing a single repeating pattern of bond impressions and unbonded area. Using the polygon drawing tool manually outline four individual bond impressions around the perimeter of the unbonded area (see example in Fig. 4B). From "View" select "Histogram of height picture". Select the number of classes as 200 and calculate the frequency histogram. Save the bond impression height file. Returning to the height image, cancel the polygon markings drawn on the bond impressions. Next, using the polygon drawing tool, manually outline the unbonded area surrounded by the bond impressions (see example in Fig. 4A). Once again, from "View" select "Histogram of height picture". Select the number of classes as 200 and calculate the frequency histogram. Save the unbonded area histogram file.

Open the histogram file of the bond impressions, determine the height range value at, or nearest to 50%. Record bond impression height to the nearest 1 micrometer. Open the histogram file of the unbonded area, determine the height range value at, or nearest to 90%. Record the unbonded area height to the nearest 1 micrometer. Measured Height is calculated as follows:

$$\text{Measured Height} = \text{unbonded area height} - \text{bond impression height}$$

Measured Height is measured at two separate ROI's for each of the specimens (*i.e.*, from front of article and back of article) to give four measures per test article. A total of three test articles are analyzed in like fashion. Calculate the Average and standard deviation for all twelve Measured Heights and report to the nearest 1 micrometer.

Tensile Strength Measurement Method

Tensile Strength is measured on a constant rate of extension tensile tester with computer interface (a suitable instrument is the MTS Alliance using Testworks 4.0 Software, as available from MTS Systems Corp., Eden Prairie, MN) using a load cell for which the forces measured are within 10% to 90% of the limit of the cell. Both the movable (upper) and stationary (lower) pneumatic jaws are fitted with rubber faced grips, wider than the width of the test specimen. All testing is performed in a conditioned room maintained at about 23°C $\pm$ 2 C and about 50% $\pm$ 2% relative humidity.

To obtain the specimen for CD tensile, lay the sample flat on a bench, body facing surface downward, and measure the total longitudinal length of the article. Note a site 25% of the total length from the front waist of the article along the longitudinal axis and a second site, 25% of the total length from the back waist of the article. Carefully remove the nonwoven outer cover from the garment-facing side of the article. A cryogenic spray, such as Cyto-Freeze (obtained from Control Company, Houston, TX), may be used to separate the nonwoven outer cover from the underlying film layer. Cut a specimen, with a die or razor knife, which is 50.8 mm wide along the longitudinal axis of the sheet and least 101.6 mm long along the lateral axis of the sheet, centered at each of the sites identified above.

In like fashion prepare MD tensile specimens from a second set of identical samples. Here, after removing the nonwoven outer cover, cut a specimen, with a die or razor knife, which is at least 101.6 mm wide along the longitudinal axis of the sheet and 50.8 mm long along the lateral axis of the sheet, centered at each of the sites identified above. Precondition both CD and MD specimens at about 23 °C $\pm$ 2 C° and about 50% $\pm$ 2% relative humidity for 2 hours prior to testing.

For analyses, set the gage length to 50.8 mm. Zero the crosshead and load cell. Insert the specimen into the upper grips, aligning it vertically within the upper and lower jaws and close the upper grips. Insert the specimen into the lower grips and close. The specimen should be under enough tension to eliminate any slack, but less than 0.05 N of force on the load cell.

Program the tensile tester to perform an extension test, collecting force and extension data at an acquisition rate of 50 Hz as the crosshead raises at a rate of 100 mm/min until the specimen breaks. Start the tensile tester and data collection. Program the software to record Peak Force (gf) from the constructed force (gf) verses extension (mm) curve. Calculate tensile strength as:

$$\text{Tensile Strength} = \text{Peak Force (gf)} / \text{width of specimen (cm)}$$

Analyze all CD tensile specimens. Record Tensile Strength to the nearest 1 gf/cm. Analyses are performed on specimens from the two sites on the article. A total of five test articles are analyzed in like fashion. Calculate and report the average and standard deviation of Tensile Strength to the nearest 1 gf/cm for all ten measured CD specimens.

Next run all MD tensile Specimens. Record Tensile Strength to the nearest 1 gf/cm. Analyses are performed on specimens from the two sites on the article. A total of five test articles are analyzed in like fashion. Calculate and report the average and standard deviation of Tensile Strength to the nearest 1 gf/cm for all ten measured MD specimens.

Image Analysis of Bond Impressions

Area and distance measurements are performed on images generated using a flat bed scanner capable of scanning at a resolution of at least 4800 dpi in reflectance mode (a suitable scanner is the Epson Perfection V750 Pro, Epson, USA). Analyses are performed using ImageJ software (Vs. 1.43u, National Institutes of Health, USA) and calibrated against a ruler certified by NIST.

To obtain the specimen, lay the sample flat on a bench, body facing surface downward, and measure the total longitudinal length of the article. Note a site 33% of the total length from the front waist of the article along the longitudinal axis and a second site, 33%of the total length from the back waist of the article. Carefully remove the nonwoven outer cover from the garment-facing side of the article. A cryogenic spray, such as Cyto-Freeze (obtained from Control Company, Houston, TX), may be used to separate the nonwoven from the underlying film layer. Cut a piece 80 mm by 80 mm centered at each site identified above. Precondition samples at about 23 °C $\pm$ 2 C° and about 50% $\pm$ 2% relative humidity for 2 hours prior to testing.

Place the specimen on the flat bed scanner, body side surface facing upward, with the ruler directly adjacent. Placement is such that the dimension corresponding to the MD of the nonwoven is parallel to the ruler. A black backing is placed over the specimen and the lid to the scanner is closed. Acquire an image composed of the nonwoven and ruler at 4800 dpi in reflectance mode in 8 bit grayscale and save the file. Open the image file in ImageJ and perform a linear calibration using the imaged ruler. Reference will be made to Fig. 3B as an example of a repeating pattern of bond impressions. These measures are equally applicable to other bond shapes and repeating bond patterns.

Average Individual Bond Area

Enlarge a ROI such that edges of the bond impression can be clearly determined. With the area tool, manually trace the perimeter of a bond. Calculate and record the area to the nearest 0.001 mm$^2$. Repeat for a total of ten non-adjacent bonds randomly selected across the total specimen. Measurements are made on both specimens from each article. A total of three identical articles are measured for each sample set. Calculate the average and standard deviation of all 60 bond area measurements and report to the nearest 0.001 mm$^2$.

Bond Path/Bond Length Ratio

Identify a single, complete repeating series of bond impressions forming a path and enlarge the image such that the repeating series fills the field of view. Draw a line along the path that connects and extends through all bond impressions in the series (*e.g.*, Fig 3B, line 105). Measure the dimensions along the line that are included within the bond impressions (*e.g.* in Fig. 3B, $D_1$, $D_2$, $D_3$). Next, measure the distance of the line segment from the leading edge of the first bond impression in the repeating series to leading edge of the first bond impression in the next adjacent series along the line segment (*e.g.* in Fig. 3B, $D_o$). Calculate the sum of the lengths of the bonds along the line segment (*e.g.* $D_1 + D_2 + D_3$), divided by the length of the line segment, (*e.g.* $D_o$), x 100%. Record the Bond Path / Bond Length Ratio to the nearest 0.001. Repeat for a total of five non-adjacent ROI's randomly selected across the total specimen. Measurements are made on both specimens from each article. A total of three identical articles are measured for each sample set. Calculate the average and standard deviation of all 60 bond Path/Length Ratio measurements and report to the nearest 0.001 units. Note for irregularly-shaped bond impressions forming a repeating series, locate a line so as to find the maximum sum of the lengths of the bond impressions measurable therealong.

Bond Area Percentage

Identify a single repeat pattern of bond impressions and unbonded areas and enlarge the image such that the repeat pattern fills the field of view. In ImageJ draw a box that encompasses the repeat pattern. For the example shown in Fig. 3B, this would be a box, $W_{S2}$ wide and $L_{S2}$ long. Note, in the example shown in Fig. 3B, the shared bond impressions at the corners are divided in half along the longitudinal or lateral direction as appropriate. Calculate area of the box and record to the nearest 0.01 mm$^2$. Next, with the area tool, trace the individual bond impressions or portions thereof entirely within the box and calculate the areas of all bond impressions or portions thereof that are within the box. Record to the nearest 0.01 mm$^2$. Calculate as follows:

$$\text{Percent Bond Area} = (\text{Sum of areas of bond impressions within box}) / (\text{area of box}) \text{ x } 100\%$$

Repeat for a total of five non-adjacent ROI's randomly selected across the total specimen. Record as Percent Bond Area to the nearest 0.01%. Measurements are made on both specimens from each article. A total of three identical articles are measured for each sample set. Calculate the average and standard deviation of all 30 of the percent bond area measurements and report to the nearest 0.001 units.

Stiffness

Stiffness of the nonwoven outer cover was measured in accordance with ASTM D6828-02. For analysis a 76.2 mm by 76.2 mm square specimen was used instead of the 100 mm by 100 mm specimen recited in the standard.

To obtain the specimen, lay the sample flat on a bench, body facing surface downward, and measure the total longitudinal length of the article. Note a site 25% of the total length from the front waist of the article along the longitudinal axis and a second site, 25% of the total length from the back waist of the article. Carefully remove the nonwoven outer cover from the garment-facing side of the article. A cryogenic spray, such as Cyto-Freeze (obtained from Control Company, Houston, TX), may be used to separate the nonwoven from the underlying film layer. Cut a piece 76.2 mm by 76.2 mm centered at each site identified above. Precondition samples at about 23 °C $\pm$ 2 C° and about 50% $\pm$ 2% relative humidity for 2 hours prior to testing.

Stiffness measurements are made on both specimens from each article. A total of three identical articles are measured for each sample set. Calculate the average and standard deviation of the six Total Stiffness results and report to 0.01g.

## Claims

1. A nonwoven web (21), formed of spunlaid fibers comprising a first polyolefin, a second polyolefin, and a softness enhancer additive, wherein said composition used to make said fibers comprises between 5% and 25% by weight of said second polyolefin and between 0.05% to 1 % softness enhancer additive by weight of said fibers; wherein said softness enhancer additive comprises at least one of oleamide, erucamide, and or stearamide; and wherein the second polyolefin is a propylene copolymer and is a different polyolefin than the first polyolefin, wherein the nonwoven web (21) is impressed with a first pattern of bond impressions (100a), the first pattern of bond impressions (100a) defining a second pattern of unbonded raised regions (101a), and the nonwoven web (21) has undergone a hydroengorgement process following a calendar bonding process.

2. The nonwoven web (21) of claim 1 wherein substantially all of the spunlaid fibers are monocomponent fibers.

3. The nonwoven web (21) of either of claims 1 or 2 wherein the first pattern of bond impressions comprises at least two adjacent, parallel, straight paths defined by the bond impressions, wherein a line (105) exists along each path, along which there are bonded lengths (D1, D2, D3) separated by unbonded lengths, and the ratio of total bonded length to total unbonded length (Bond Length Ratio) is at least 35% but less than 100%.

4. The nonwoven web (21) of any of the preceding claims wherein the bond impressions (100a) constitute a Bond Area Percentage of at least 10%.

5. The nonwoven web (21) of claim 4 wherein the Bond Area Percentage is no more than 20%.

6. The nonwoven web (21) of any of the preceding claims wherein said second polyolefin is a propylene ethylene copolymer with between 5% and 35% of ethylene units.

7. The nonwoven web (21) of any of the preceding claims wherein said calendering bonds provide said nonwoven (21) material with a first textured surface and a second surface opposite said first surface.

8. The nonwoven web (21) of any of the preceding claims wherein said first polyolefin comprises polypropylene and said second polyolefin comprises an elastomeric polypropylene.

9. The nonwoven web (21) of any of the preceding claims wherein said nonwoven (21) material has a basis weight of between 6 $g/m^2$ and 50 $g/m^2$.

10. The nonwoven web (21) of claim 9 wherein said nonwoven (21) material has a caliper of between 0.15 mm and 1 mm.

11. The nonwoven web (21) of any of the preceding claims, wherein said propylene copolymer has a triad tacticity of three propylene units, as measured by 13C NMR according to description of at least about 75%, at least about 80%, at least about 82%, at least about 85%, or at least about 90%

12. The nonwoven web (21) of any of the preceding claims, wherein the softness enhancer additive comprises chemical compounds having a nitrogen bond to organic chain, preferably being organic amine or amide.

13. An absorbent article comprising a topsheet (18), a backsheet (20) and an absorbent core (14) disposed therebetween, the absorbent article having a component comprising the nonwoven web (21) of any of the preceding claims.

## Patentansprüche

1. Vliesbahn (21), die aus spinngelegten Fasern gebildet wird, umfassend ein erstes Polyolefin, ein zweites Polyolefin, und einen Weichheitsverstärker-Zusatzstoff, wobei die zur Herstellung der Fasern verwendete Zusammensetzung zwischen 5 und 25 Gew.-% des zweiten Polyolefins und zwischen 0,05 bis 1 Gew.-% Weichheitsverstärker-Zusatzstoff, bezogen auf das Gewicht der Fasern, umfasst; wobei der Weichheitsverstärker-Zusatzstoff mindestens eines von Oleamid, Erucamid, und/oder Stearamid umfasst; und

wobei das zweite Polyolefin ein Propylen-Copolymer ist, und ein von dem ersten Polyolefin verschiedenes Polyolefin ist,

wobei die Vliesbahn (21) mit einem ersten Muster von Verbunddrucken (100a) bedruckt ist, wobei das erste Muster von Verbunddrucken (100a) ein zweites Muster ungebundener erhöhter Bereiche (101a) definiert, und mit der Vliesbahn (21) ein Wasserstrahlverfestigungsverfahren und anschließend ein Kalandrierbindungsverfahren durchgeführt wurden.

2. Vliesbahn (21) nach Anspruch 1, wobei im Wesentlichen alle spinngelegten Fasern Einkomponentenfasern sind.

3. Vliesbahn (21) nach einem der Ansprüche 1 oder 2, wobei das erste Muster von Verbunddrucken mindestens zwei nebeneinanderliegende, parallele, gerade, durch die Verbunddrucke definierte Pfade umfasst, wobei eine Linie (105) entlang jedes Pfads existiert, entlang derer gebundene Längen (D1, D2, D3) vorhanden sind, die durch ungebundene Längen getrennt sind, und das Verhältnis der gebundenen Länge insgesamt zur ungebundenen Länge insgesamt (Bindungslängenverhältnis) mindestens 35 %, jedoch weniger als 100 % beträgt.

4. Vliesbahn (21) nach einem der vorstehenden Ansprüche, wobei die Verbunddrucke (100a) eine prozentuale Bindungsfläche von wenigstens 10 % ausmachen.

5. Vliesbahn (21) nach Anspruch 4, wobei die prozentuale Bindungsfläche nicht mehr als 20 %. beträgt.

6. Vliesbahn (21) nach einem der vorstehenden Ansprüche, wobei das zweite Polyolefin ein Propylenethylen-Copolymer mit zwischen 5 und 35 % Ethyleneinheiten ist.

7. Vliesbahn (21) nach einem der vorstehenden Ansprüche, wobei die Kalandrierbindungen das Vlies (21)-Material mit einer ersten strukturierten Oberfläche und einer zweiten Oberfläche gegenüber der ersten Oberfläche bereitstellen.

8. Vliesbahn (21) nach einem der vorstehenden Ansprüche, wobei das erste Polyolefin Polypropylen umfasst und das zweite Polyolefin ein elastomeres Polypropylen umfasst.

9. Vliesbahn (21) nach einem der vorstehenden Ansprüche, wobei das Vlies (21)-Material ein Flächengewicht von zwischen 6 und 50 g/m$^2$ aufweist.

10. Vliesbahn (21) nach Anspruch 9, wobei das Vlies (21)-Material eine Dicke von zwischen 0,15 und 1 mm aufweist.

11. Vliesbahn (21) nach einem der vorstehenden Ansprüche, wobei das Proypylen-Copolymer eine Dreier-Taktizität von drei Propyleneinheiten, wie durch 13C-NMR gemäß Beschreibung gemessen, von mindestens etwa 75 %, mindestens etwa 80 %, mindestens etwa 82 %, mindestens etwa 85 %, oder mindestens etwa 90 % aufweist.

12. Vliesbahn (21) nach einem der vorstehenden Ansprüche, wobei der Weichheitsverstärker-Zusatzstoff chemische Verbindungen mit einer Stickstoffbindung zur organischen Kette umfasst, die vorzugsweise organisches Amin oder Amid darstellen.

13. Absorptionsartikel, umfassend eine Oberschicht (18), eine Unterschicht (20) und einen dazwischen angeordneten Absorptionskern (14), wobei der Absorptionsartikel eine Komponente aufweist, die die Vliesbahn (21) nach einem der vorstehenden Ansprüche umfasst.

**Revendications**

1. Nappe non tissée (21), formée de fibres filées-liées comprenant une première polyoléfine, une deuxième polyoléfine et un additif amplificateur de douceur, dans laquelle ladite composition utilisée pour fabriquer lesdites fibres comprend entre 5 % et 25 % en poids de ladite deuxième polyoléfine et entre 0,05 % et 1 % d'additif amplificateur de douceur en poids desdites fibres ; dans laquelle ledit additif amplificateur de douceur comprend au moins l'un parmi l'oléamide, l'érucamide et/ou le stéaramide ; et

dans laquelle la deuxième polyoléfine est un copolymère de propylène et est une polyoléfine différente de la première polyoléfine,

où la nappe non tissée (21) est imprimée avec un premier motif d'impressions de liaison (100a), le premier motif d'impressions de liaison (100a) définissant un deuxième motif de régions relevées non liées (101a) et la nappe non tissée (21) a subi un processus d'hydro-engorgement à la suite d'un processus de liaison par calandrage.

2. Nappe non tissée (21) selon la revendication 1, dans laquelle essentiellement toutes les fibres filées-liées sont des fibres à monocomposant.

3. Nappe non tissée (21) selon la revendication 1 ou la revendication 2, dans laquelle le premier motif d'impressions de liaison comprend au moins deux trajectoires linéaires adjacentes, parallèles définies par les impressions de liaison, dans laquelle une ligne (105) existe le long de chaque trajectoire, le long de laquelle il y a des longueurs liées (D1, D2, D3) séparées par des longueurs non liées, et le rapport de la longueur liée totale à la longueur non liée totale (rapport de longueurs de liaison) est d'au moins 35 %, mais inférieur à 100 %.

4. Nappe non tissée (21) selon l'une quelconque des revendications précédentes, dans laquelle les impressions de liaison (100a) constituent un pourcentage de surface de liaison d'au moins 10 %.

5. Nappe non tissée (21) selon la revendication 4, dans laquelle le pourcentage de surface de liaison n'est pas supérieur à 20 %.

6. Nappe non tissée (21) selon l'une quelconque des revendications précédentes, dans laquelle ladite deuxième polyoléfine est un copolymère de propylène/éthylène avec entre 5 % et 35 % de motifs éthylène.

7. Nappe non tissée (21) selon l'une quelconque des revendications précédentes, dans laquelle lesdites liaisons par calandrage fournissent audit matériau non tissé (21) une première surface texturée et une deuxième surface opposée à ladite première surface.

8. Nappe non tissée (21) selon l'une quelconque des revendications précédentes, dans laquelle ladite première polyoléfine comprend du polypropylène et ladite deuxième polyoléfine comprend un polypropylène élastomère.

9. Nappe non tissée (21) selon l'une quelconque des revendications précédentes, dans laquelle ledit matériau non tissé (21) a une masse surfacique comprise entre 6 g/m$^2$ et 50 g/m$^2$.

10. Nappe non tissée (21) selon la revendication 9, dans laquelle ledit matériau non tissé (21) a une épaisseur comprise entre 0,15 mm et 1 mm.

11. Nappe non tissée (21) selon l'une quelconque des revendications précédentes, dans laquelle ledit copolymère de propylène a une tacticité de triade de trois motifs propylène, telle que mesurée par 13C RMN selon la description d'au moins environ 75 %, au moins environ 80 %, au moins environ 82 %, au moins environ 85 % ou au moins environ 90 %

12. Nappe non tissée (21) selon l'une quelconque des revendications précédentes, dans laquelle l'additif amplificateur de douceur comprend des composés chimiques ayant une liaison azote à la chaîne organique, étant de préférence une amine ou un amide organique.

13. Article absorbant comprenant une feuille de dessus (18), une feuille de fond (20) et une âme absorbante (14) disposée entre elles, l'article absorbant ayant un composant comprenant la nappe non tissée (21) selon l'une quelconque des revendications précédentes.

Fig. 1A

Fig. 1B

Fig. 2A

Fig. 2B

Fig. 3A

Fig. 3B

Fig. 4A

Fig. 4B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2006057921 A
- WO 2014044235 A
- WO 2012130414 A
- WO 2012024576 A
- US 5246433 A, Hasse
- US 5569234 A, Buell
- US 6120487 A, Ashton
- US 6120489 A, Johnson
- US 4940464 A, Van Gompel
- US 5092861 A, Nomura
- US 20030233082 A1
- US 5897545 A, Kline
- US 5957908 A, Kline
- US 3860003 A
- US 5151092 A
- US 5554145 A
- US 6004306 A
- US 5037416 A
- US 5269775 A
- WO 9516746 A
- US 5571096 A, Dobrin
- US 5266392 A
- US 058376 A
- US 14058398 B
- US 14058479 B
- US 5370764 A
- US 20130253461 A
- US 20060057921 A
- US 20080312624 A

**Non-patent literature cited in the description**

- **H. N. CHENG.** *MACROMOLECULES 1950,* 1984, vol. 17
- **VERSTATE et al.** *MACROMOLECULES 3360,* 1988, vol. 21